# EUROPEAN PATENT SPECIFICATION

(11) **EP 4 065 136 B1**
(45) Date of publication and mention of the grant of the patent: **14.08.2024**
(21) Application number: 20819848.1
(22) Date of filing: 26.11.2020
(51) Int. Cl.: A61P 35/00, C07K 14/435, A61K 39/00

(54) **PHAGOCYTOSED SELF-ASSEMBLING PROTEINS**
PHAGOZYTOSIERTE SELBSTASSEMBLIERENDE PROTEINE
PROTÉINES PHAGOCYTÉES À AUTO-ASSEMBLAGE

(30) Priority: 28.11.2019 GB 201917381
(43) Date of publication of application: 05.10.2022
(73) Proprietor: Cell Guidance Systems Limited, Cambridge, Cambridgeshire CB22 3AT (GB)
(72) Inventor: JONES, Michael, Cambridge, Cambridgeshire CB22 3AT (GB); PERNSTICH, Christian, Cambridge, Cambridgeshire CB22 3AT (GB); WENDLER, Astrid, Cambridge, Cambridgeshire CB22 3AT (GB); JAMES, Nicholas, Cambridge, Cambridgeshire CB22 3AT (GB)
(74) Representative: Secerna LLP
(86) International application number: PCT/GB2020/053020
(87) International publication number: WO 2021/105688

(56) References cited:
- WO-A2-2010/141953
- HAJIME MORI ET AL: "Immobilization of Bioactive Fibroblast Growth Factor-2 into Cubic Proteinous Microcrystals ( Bombyx mori Cypovirus Polyhedra) That Are Insoluble in a Physiological Cellular Environment", JOURNAL OF BIOLOGICAL CHEMISTRY, vol. 282, no. 23, 12 April 2007 (2007-04-12), pages 17289 - 17296, XP055489427, ISSN: 0021-9258, DOI: 10.1074/jbc.M608106200
- MANOJ S. NAIR ET AL: "Cry Protein Crystals: A Novel Platform for Protein Delivery", PLOS ONE, vol. 10, no. 6, 1 June 2015 (2015-06-01), pages 1 - 16, XP055463443, DOI: 10.1371/journal.pone.0127669
- YANG ZAOFENG ET AL: "Targeted delivery of antimicrobial peptide by Cry protein crystal to treat intramacrophage infection", BIOMATERIALS, ELSEVIER, AMSTERDAM, NL, vol. 217, 15 June 2019 (2019-06-15), XP085743163, ISSN: 0142-9612, [retrieved on 20190615], DOI: 10.1016/J.BIOMATERIALS.2019.119286

## Description

### Field of the invention

The present invention relates to self-assembling proteins that are capable of being used to deliver cargo proteins to phagocytic cells, and the subsequent delivery and utility of these self-assembling proteins by the phagocytic cells at sites of disease. In particular, the invention relates *inter alia* to professional phagocytic cells comprising polyhedrin protein crystals, wherein the crystals themselves encapsulate therapeutic and/or diagnostic proteins. The invention also provides pharmaceutical compositions comprising these cells for use in medicine.

### Background to the invention

Proteins, including antibodies, growth factors, hormones, enzymes and vaccines can be used therapeutically. For example, growth factors such as IL-2 have been shown to have therapeutic value in cancer (Andersen et al, 2016). However, upon systemic injection, proteins can be rapidly cleared and require doses that generate significant toxic side effects to non-target cells. For primary tumours, it may be possible to inject proteins directly into the tumour mass, but protein instability, particularly for growth factors, still creates challenges with this approach. Although therapy may be feasible by direct injection into the tumour mass, delivery of growth factors and other proteins to any widely disseminated disease cannot practically be achieved this way.

Phagocytic cells engulf microscopic particles through an active phagocytic process. A wide variety of different phagocytic cells types are present in mammals. Phagocytic cells are key to the immune system's response to infection and disease. For example, macrophages are specialized cells of the innate and adaptive immune systems which play a key role in defending against foreign pathogens, healing wounds, and regulating tissue homeostasis. Responding to subtle changes in the environment, macrophages are activated, also referred to as "polarized", to a pro-inflammatory M1 or an anti-inflammatory M2 phenotype. Activation can be transient and is non-binary with high levels of cell plasticity.

Stimuli such as IFN-γ, LPS and GM-CSF result in polarization to the M1 phenotype, which is characterized by the secretion of pro-inflammatory cytokines (e.g. IL-6, IL-1β and IL-12) and chemokines (e.g. CCL5, CCL10 and CXL9), and the release of reactive oxygen and nitrogen intermediates - the "classical" pathogen killing Th1 response. In contrast, M2 macrophages stimulated by the cytokines IL-4 and IL-13, secrete anti-inflammatory cytokines (e.g. IL-10 and TGF-β) and chemokines (e.g. CCL17 and CCL22).

When polarized macrophages encounter a foreign object such as microbes or other pathogens, uptake into the cell is initiated. Once phagocytosed the innate acidic phagolysosome normally efficiently degrades biological material including proteins. Subsequently, fragments of surface proteins of degraded pathogens are presented on the surface of macrophages for recognition by other cells of the immune system.

Phagocytic cells may also infiltrate diseased tissue. However, phagocytic cells may be subverted and actively contribute to the genesis of diseases such as solid cancers (van Dalen et al 2019). For example, tumor-associated macrophages (TAMs) usually express an M2 phenotype (alternatively activated subset), which leads to them performing immunosuppressive and tumor promoting functions. Reprogramming of such cells towards an M1 phenotype (classically activated subset) may suppress their pro-cancer phenotype and unleash anti-tumor immunity.

Given their intimate association with disease and their ability to infiltrate diseased tissue, the ease with which they can be harvested for ex-vivo manipulation, and their alternative states, it has been proposed that (1) the phenotype of phagocytic cells may be modulated to impact disease outcome and (2) phagocytic cells may be used as "trojan horses" to deliver a drug preferentially or specifically to an area of disease, targeting other cells whilst reducing toxic side effects to non-target tissue (Choi et al 2007).

To date, several strategies have been developed to re-purpose monocytes and macrophages to deliver drugs to sites of infection, inflammation or solid tumors (Xu et al 2018; Pang et al 2017; Rosa et al 2017). However, the fragility of cargo therapeutic proteins has confounded attempts to use them in a Trojan horse strategy to treat disease. There are numerous challenges in successfully delivering therapeutic cargo proteins using phagocytic cells such as macrophages, with toxicity to the phagocytic cell, changes to their metabolism, efficiency of uptake and digestion of cargo to name but a few.

Mori et al. J. Biol. Chem. (2007) 282: 17289-17296 concerns the production of polyhedra-immobilized growth factors. WO 2010/141953 A2 relates to use of Bacillus thuringiensis to produce regularly shaped micrometer-sized crystals of Cry insect protoxins. Nair et al PLOS ONE (2015) 10: 6, 1-16 concerns delivery agents based on Cry3Aa protein crystals that naturally form within the bacterium Bacillus thuringiensis. Zaofeng et al BIOMATERIALS (2019) 217, 9-10 relates to use of Cry3Aa crystals to deliver antimicrobial peptides.

It is an aim of some embodiments of the present invention to mitigate some of the problems identified in the prior art.

### Summary of certain embodiments of the invention

The invention relates to the use of polyhedrin protein crystals (e.g. PODS^{®} protein crystals) to deliver cargo proteins via phagocytic cells. PODS^{®} are micron scale cubic co-crystals which may contain stabilized recombinant protein cargo which is sustainably released under the activity of proteases. Certain aspects of the present invention relate to the finding that such crystals are efficiently phagocytosed into the cells and, unexpectedly, secrete their cargo in a way that retains cargo bioactivity and also modulates the behavior of nearby cells.

Certain aspects of the invention relate to the finding that polyhedrin protein crystals are efficiently taken up by macrophage cells and, against expectations, proteins incorporated into these crystals are subsequently released from the cells in a bioactive form. Moreover, the uptake of these crystals neither affects macrophage cell viability nor interferes with key macrophage function such as following chemotactic signals secreted by tumor cells or migrating through narrow spaces, both important for efficient delivery of therapeutic cargo to sites of disease.

Certain aspects of the invention relate to improved methods of delivering therapeutic cargo proteins to sites of disease by infiltrating phagocytic cells such as macrophages to increase drug efficacy and reduce off-target side effects. Alternative aspects of the invention relate to methods of delivering diagnostic cargo proteins to sites of disease by infiltrating phagocytic cells such as macrophages in order to image a disease.

Accordingly, the invention provides:
- a phagocytic cell comprising one or more polyhedrin protein crystals, wherein the crystals encapsulate one or more cargo proteins, wherein the phagocytic cell is a professional cell and the cargo protein comprises one or more therapeutic agents;
- a population comprising more than 1 × 10⁵ phagocytic cells as claimed herein;
- a package comprising a phagocytic cell (or population thereof) as claimed herein;
- a method of producing a phagocytic cell (or population thereof) as claimed herein, comprising:
   (a) contacting the one or more phagocytic cells with polyhedrin protein crystals; and
   (b) culturing the phagocytic cells under conditions which induce phagocytosis;
- a pharmaceutical composition comprising:
   (a) a phagocytic cell (or population thereof) as claimed herein, wherein the phagocytic cell comprises one or more therapeutic agents; and
   (b) a pharmaceutically acceptable carrier or diluent;
- a pharmaceutical composition as claimed herein for use in medicine;
- a pharmaceutical composition as claimed herein for use in a method of treating and/or preventing a solid cancer in a subject; and
- a phagocytic cell or population thereof for use in a method of imaging a solid cancer, wherein the phagocytic cell is a professional cell and comprises one or more polyhedrin protein crystals wherein the crystals encapsulate one or more detectable agents, or the population comprise more than 1 × 10⁵ phagocytic cells, and wherein the method comprises:
   (a) administering the phagocytic cell or the population thereof,; and
   (b) detecting the detectable agent, thereby imaging at least part of the cancer.

### Detailed description of certain embodiments

Embodiments of the invention will now be described by way of example only, and with reference to the accompanying Figures in which:

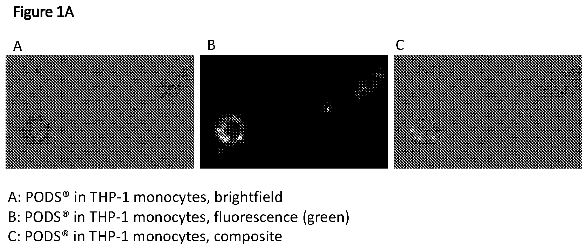

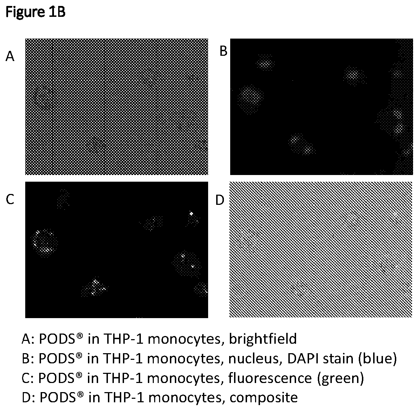

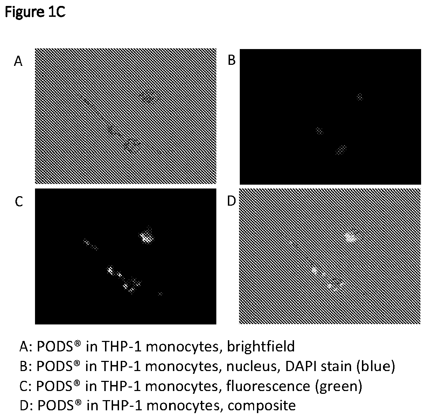

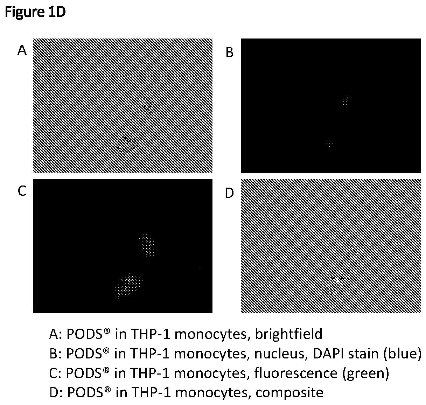

Figure 1 shows phagocytosis of PODS^{®} protein crystals into professional phagocytes. THP-1 monocytes were incubated with PODS^{®} crystals containing enhanced green fluorescent protein (eGFP) for 24 h. Cells were subsequently imaged either in a) brightfield mode (A); fluorescence mode (B); composite image (C). Or b-d) in brightfield mode (A); DAPI stained for nuclei (B); fluorescence mode (C); composite image (D). Composites show multiple cubic PODS@ eGFP crystals phagocytosed into live monocytes.

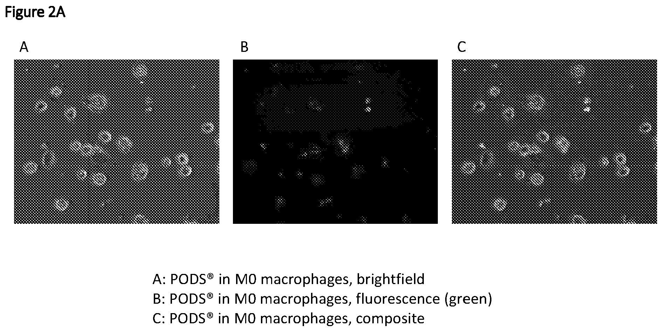

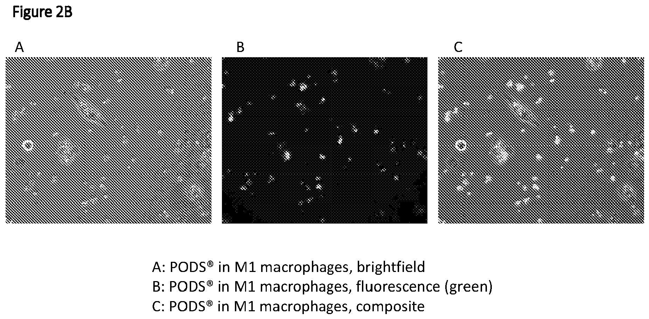

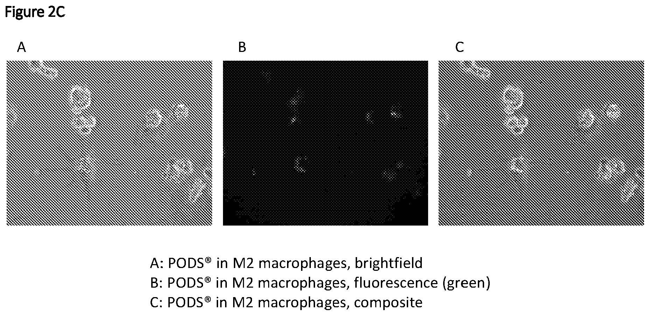

Figure 2 shows phagocytosis of PODS^{®} protein crystals into various types of professional phagocytes. THP-1 monocytes were differentiated first into M0 macrophages (a) and then further into either M1 (B) or M2 (C) macrophages. Cells of the various differentiated types were cultured with PODS^{®} eGFP for 24 h and subsequently imaged in brightfield (A) and fluorescence mode (B), respectively.

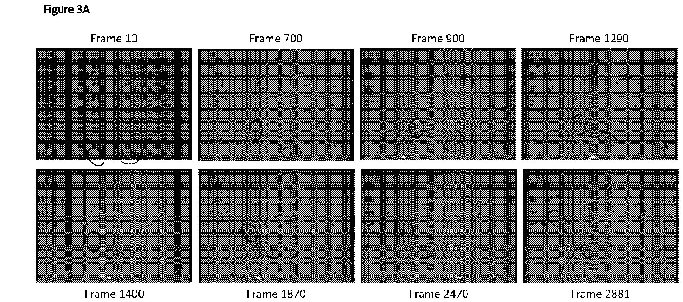

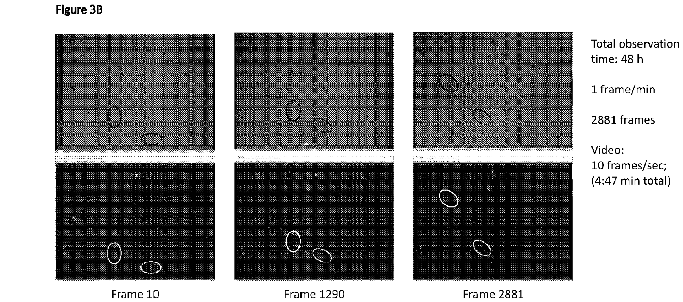

Figure 3 shows stills from a time-lapse video observing phagocytosis of PODS@ eGFP crystals. Differentiated THP-1 cells (M0 macrophages) were incubated with PODS@ eGFP crystals for 48h. Images were taken at a rate of 1 frame/min in brightfield mode and fluorescence mode. A) Brightfield mode tracking of two individual monocytes (black ovals) in the field of view over time, as indicated by frame numbers, reveals mobility of M0 macrophages as well their phagocytosis of several PODS@ crystals along that path of motion. B) Three corresponding stills of the time-lapse video, both in brightfield mode and fluorescence imaging mode, where the position of the tracked M0 macrophages are marked (black and white ovals, respectively). The initially static PODS^{®} eGFP particles move, once internalized, according to the trajectory of migrating monocytes.

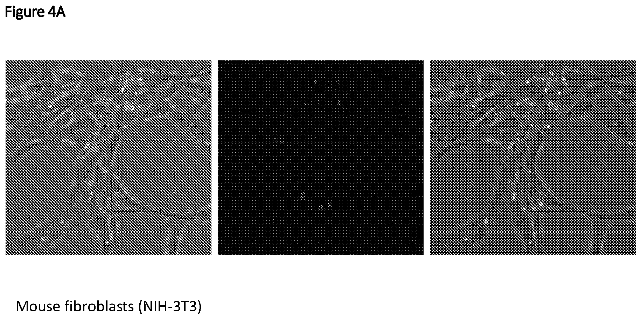

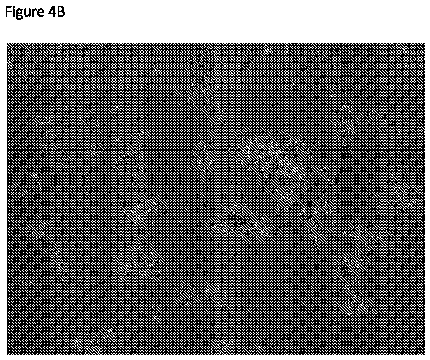

Figure 4 shows phagocytosis of PODS^{®} protein crystals into various types of non-professional phagocytes. Cells were cultured with PODS^{®} eGFP for 24 h and subsequently either imaged live (a) or fixed and imaged (b). a) Phagocytosis into mouse fibroblast cells (NIH-3T3) imaged, after a 24 h incubation period, in brightfield (A) and fluorescence mode (B), respectively.

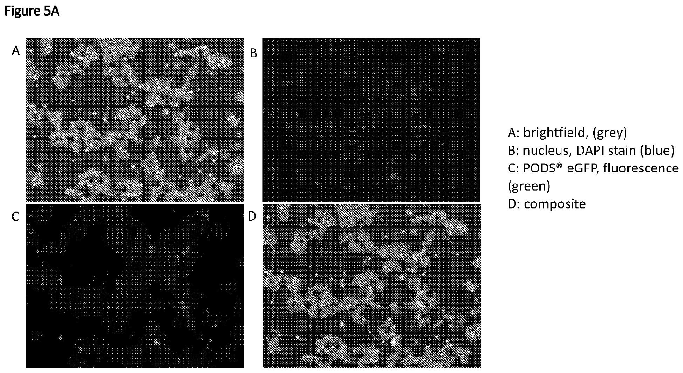

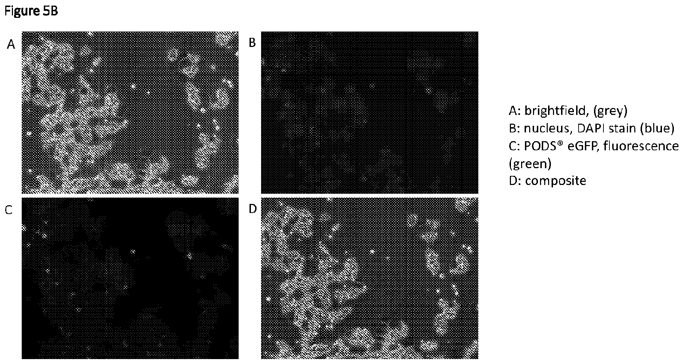

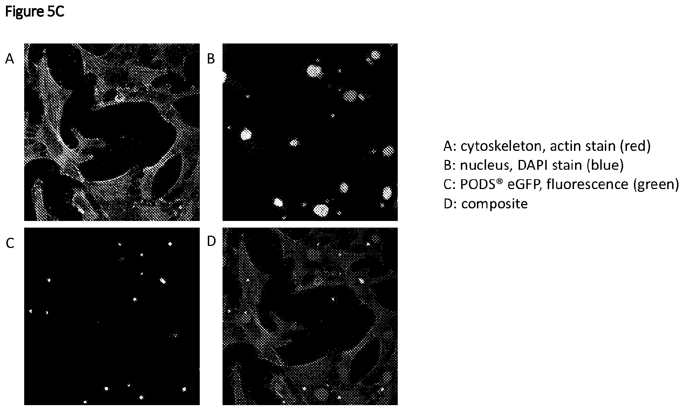

Figure 5 shows analysis of phagocytosis. PODS^{®} Empty were adhered to wells and incubated with professional phagocytes (either monocytes or M1 macrophages) for 24 h. Cells were subsequently fixed and exposed first to anti-polyhedrin primary antibody and then anti-rabbit FITC secondary antibody. Micrographs of monocytes (a) and M1 macrophages (b) with phagocytosed PODS@ Empty were taken in brightfield and fluorescence mode. c) b) immunohistochemistry (IHC) images of PODS^{®} eGFP phagocytosed into chondrocytes. PODS^{®} eGFP were adhered onto the surface of wells and incubated with chondrocytes for 24 h. Cells were then fixed and slides were either stained for the cytoskeleton with actin (A) or for nuclei with DAPI (B); fluorescence signal from PODS^{®} eGFP (C); and the composite (D).

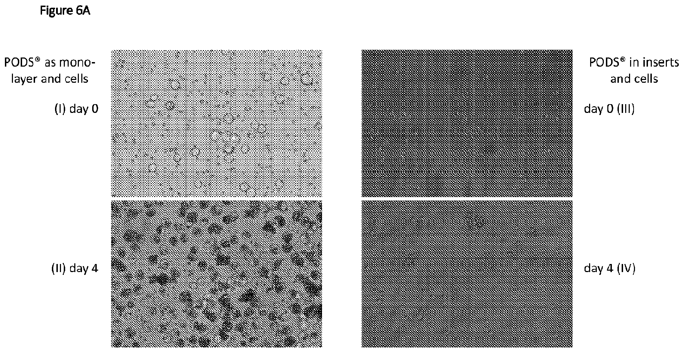

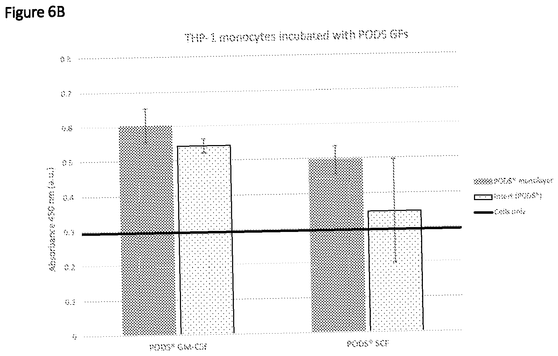

Figure 6 shows proliferation of THP-1 monocytes stimulated by PODS^{®} protein crystals. PODS@ were seeded either directly onto the well of a 24-well plate as a single crystal layer or into 24-well tissue culture inserts. Subsequently, monocytes were seeded into the wells that contained either a monolayer of PODS^{®} protein crystals; or an insert with PODS^{®} protein crystals; or an empty insert as baseline control. a) Representative brightfield micrographs of monocyte cultures with PODS^{®} seeded as monolayers in the well on day 0 (I) and day 4 (II). Micrographs from experiments with PODS@ in TC inserts show only cells in the well on both day 0 (III) as well as day 4 (IV). b) After 4 days of incubation, total cell numbers were assessed using a colorimetric assay (OrangU, Cell Guidance Systems). The horizontal line represents THP-1 cells without further supplement; plain grey bars represent results from experiments with PODS^{®} as monolayer; dotted bars shows results from PODS@ in TC inserts. Error bars depict standard deviation from the mean.

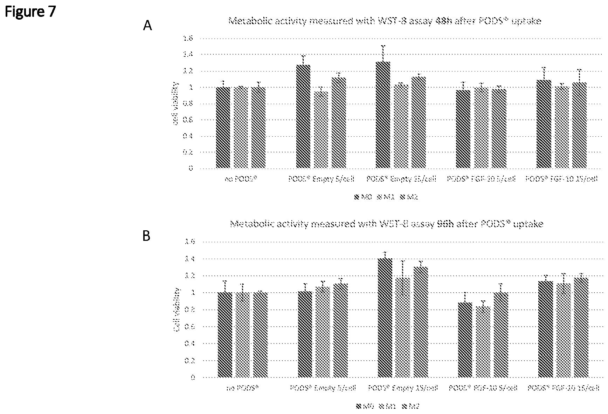

Figure 7 shows viability of macrophages after PODS@ uptake. M0, M1 and M2 macrophages were incubated with PODS^{®} Empty or PODS^{®} FGF-10 (1:5 and 1:15) for 24 h in a 96-well TC plate. Subsequently, medium was changed and viability of cells was measured 48h (A) and 96h (B) after PODS@ uptake using a colorimetric assay (Orangu^{™}, Cell Guidance Systems).

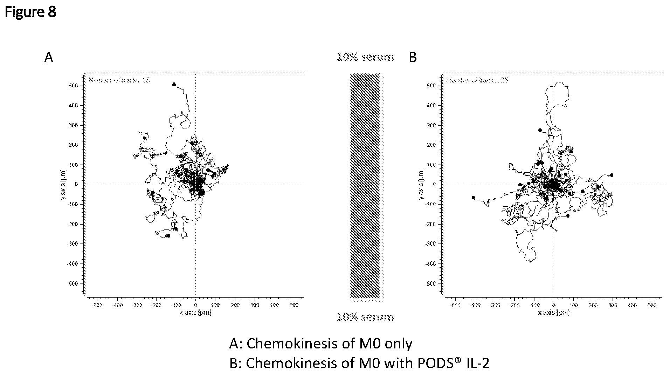

Figure 8 shows mobility of PODS^{®} IL-2 loaded macrophages. The mobility of empty (A) and PODS@ IL-2 loaded macrophages (10 PODS^{®}/cell) (B) in full growth medium was monitored using a live cell imaging system. Images were taken every 2 min for 24h and analysed using the manual tracker of Image J, and the chemotaxis and migration tool (Ibidi). 25 cells per condition were followed and their migration tracks visualised as a rose plot, in which each line represents the track of an individual cell over 24 hours. Track positions were normalized so that each track starts at the (0,0) coordinate.

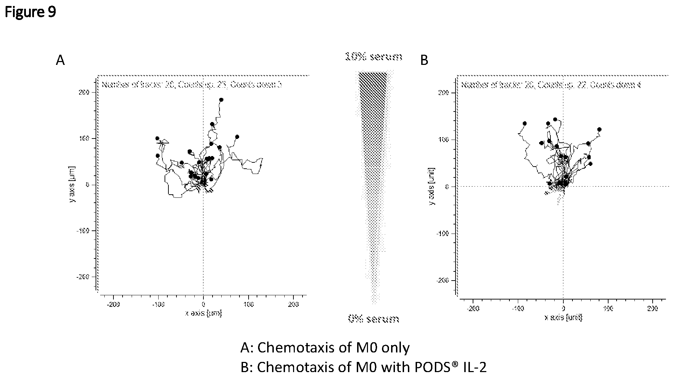

Figure 9 shows directed migration of PODS^{®} IL-2 loaded macrophages. Empty (A) and PODS@ IL-2 loaded macrophages were transferred into Chemotaxis µ-Slides (Ibidi) and tested for their ability to follow a chemoattractant gradient generated from 0-10% BCS. Images were taken every 1 min for 12 h and analysed using the manual tracker utility of Image J, and the chemotaxis and migration tool (Ibidi). 26 cells per condition were followed and their migration tracks visualised as a rose plot, in which each line represents the track of an individual cell over 12 hours. Track positions were normalized so that each track starts at the (0,0) coordinate. Tracks of cells migrated towards the 10% BCS source are shown in black (up), whereas track of cells migrated away from the BCS source are shown in red (down).

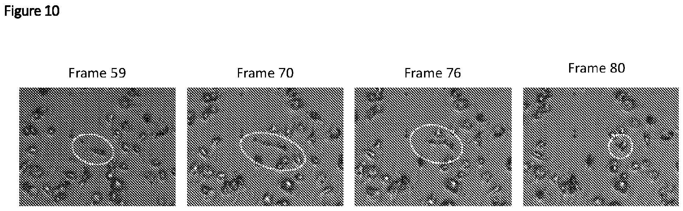

Figure 10 shows mobility of PODS@ eGFP loaded bone marrow-derived mouse macrophages. BMDMs were incubated with PODS^{®} eGFP for 24 h and then monitored using a live cell imaging system at 1 frame/min. The dashed white circle follows the movement and contraction of a macrophage over 21 frames equivalent to 21 min.

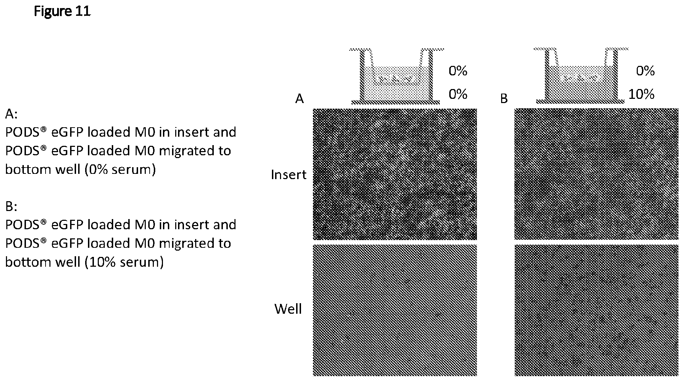

Figure 11 shows directed migration of PODS@ eGFP loaded macrophages through 8 µm pores. PODS^{®} eGFP loaded macrophages (5 PODS@ /cell) in serum-free medium were transferred into 24-well inserts with 8 µm pores. The bottom well was filled with either RPMI-1640 only (A) or RPMI-1640 supplemented with 10% FBS (B). The upper images show the inserts and the lower images show the bottom well after 24h of incubation.

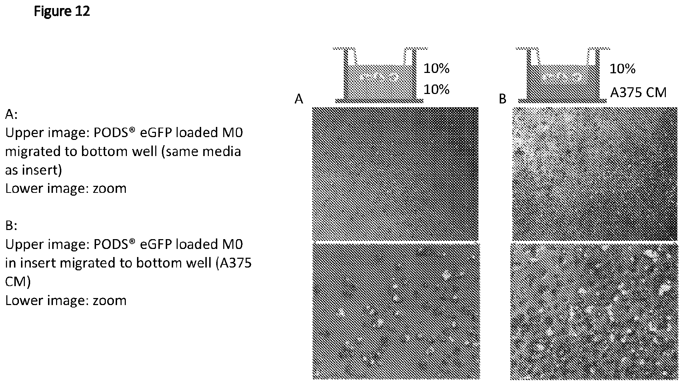

Figure 12 shows PODS@ eGFP loaded macrophages follow chemical signals from melanoma cell line through 8 µm pores. PODS^{®} eGFP loaded macrophages (5 PODS@ /cell) in full growth medium were transferred into 24-well inserts with 8 µm pores. The well was filled with either full growth medium (A) or medium that was conditioned with A375 cells for 3 days (B). Top images are representative and show the number of macrophages migrated into the well after 24 h of subsequent incubation; lower images display enlarged sections.

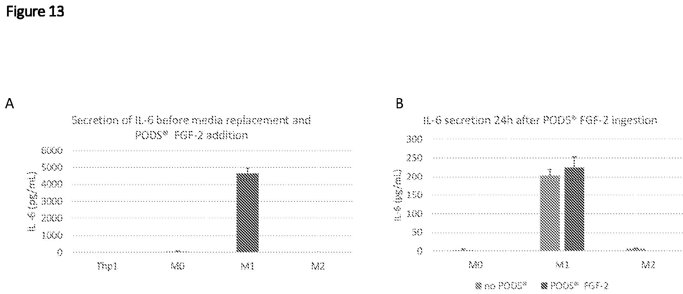

Figure 13 shows endogenous secretion of IL-6. Medium of THP-1, M0, M1 and M2 was collected after polarisation and tested for the presence of IL-6 by ELISA (A). The same cells were then washed and incubated with PODS^{®} FGF-2 for 24 h in full growth medium. The medium was collected and analysed for the presence of IL-6 by ELISA (B).

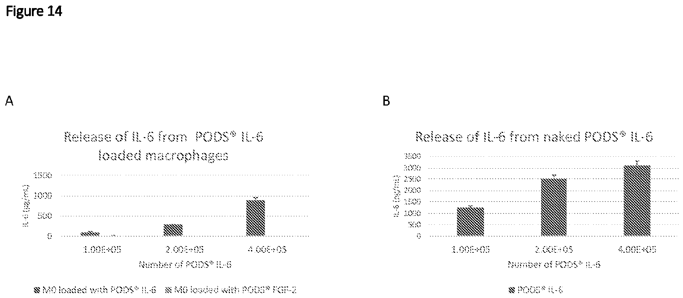

Figure 14 shows release of IL-6 from PODS@ IL-6 loaded Macrophages. (A) M0 were loaded with 5, 10 or 20 of either PODS^{®} IL-6 or PODS^{®} FGF-2 per cell. Cells were washed and incubated in full growth medium for 4 d. Medium was collected and tested for the presence of IL-6 in the medium. (B) The same amounts of PODS^{®} IL-6 as before were spun down to the bottom of a 96-well plate and incubated for 4 d in full growth medium. The medium was collected and levels of IL-6 measured by ELISA.

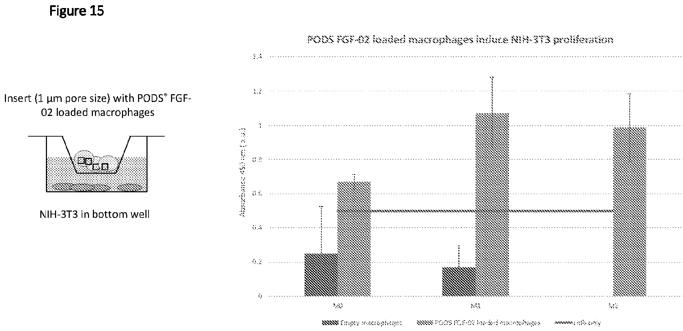

Figure 15 shows bioactivity of FGF-2 released from PODS^{®} FGF-2 loaded macrophages. Unloaded and FGF-2 loaded macrophages (M0, M1 and M2 with 10 PODS^{®}/cell) in TC inserts were incubated with FGF-2 reactive NIH-3T3 cells under serum-free conditions for 4 d. Proliferation of NIH-3T3 cells was measured using a colorimetric assay (Orangu^{™}, Cell Guidance Systems).

The practice of embodiments of the present invention employs, unless otherwise indicated, conventional techniques of chemistry, molecular biology, pharmaceutical formulation, pharmacology and medicine, which are within the skill of those working in the art.

Most general chemistry techniques can be found in Comprehensive Heterocyclic Chemistry IF (Katritzky et al., 1996, published by Pergamon Press); Comprehensive Organic Functional Group Transformations (Katritzky et al., 1995, published by Pergamon Press); Comprehensive Organic Synthesis (Trost et al,. 1991, published by Pergamon); Heterocyclic Chemistry (Joule et al. published by Chapman & Hall); Protective Groups in Organic Synthesis (Greene et al., 1999, published by Wiley-Interscience); and Protecting Groups (Kocienski et al., 1994).

Most general molecular biology techniques can be found in Sambrook et al, Molecular Cloning, A Laboratory Manual (2001) Cold Harbor-Laboratory Press, Cold Spring Harbor, N.Y. or Ausubel et al., Current Protocols in Molecular Biology (1990) published by John Wiley and Sons, N.Y.

Most general pharmaceutical formulation techniques can be found in Pharmaceutical Preformulation and Formulation (2nd Edition edited by Mark Gibson) and Pharmaceutical Excipients: Properties, Functionality and Applications in Research and Industry (edited by Otilia M Y Koo, published by Wiley).

Most general pharmacological techniques can be found in A Textbook of Clinical Pharmacology and Therapeutics (5th Edition published by Arnold Hodder).

Most general techniques on the prescribing, dispensing and administering of medicines can be found in the British National Formulary 72 (published jointly by BMJ Publishing Group Ltd and Royal Pharmaceutical Society).

Unless defined otherwise, all technical and scientific terms used herein have the same meaning as commonly understood by one of ordinary skill in the art to which this disclosure belongs. For example, the Concise Dictionary of Biomedicine and Molecular Biology, Juo, Pei-Show, 2nd ed., 2002, CRC Press; The Dictionary of Cell and Molecular Biology, 3rd ed., Academic Press; and the Oxford University Press, provide a person skilled in the art with a general dictionary of many of the terms used in this disclosure. For chemical terms, the skilled person may refer to the International Union of Pure and Applied Chemistry (IUPAC).

Units, prefixes and symbols are denoted in their Système International d'Unités (SI) accepted form. Numeric ranges are inclusive of the numbers defining the range.

### Phagocytic cells

The invention provides one or more phagocytic cells as claimed herein, wherein the phagocytic cells are professional cells.

Typically, the phagocytic cells are mammalian cells. For example, the phagocytic cells may be murine or human cells. Professional phagocytic cells of the invention include monocytes, macrophages and glial cells.

In certain embodiments, the phagocytic cells of the invention are obtained from monocytes. For example, the phagocytic cells may be obtained from immortalized monocyte-like cell lines (e.g. THP-1 ATCC).

In certain embodiments, pre-existing phagocytic cells are isolated from body cavity lavages (alveolar, peritoneal) of resident or elicited monocytes (Zhang et al, 2008).

In certain embodiments, the phagocytic cells of the invention are obtained by differentiating monocytes from blood or extracted from bone marrow. For example, the phagocytic cells may be obtained from peripheral blood mononuclear cells (PBMCs).

In certain embodiments, monocytes may be differentiated by supplementing culture media with growth factors or signaling molecules such as cytokines. For example, the culture media may be supplemented with macrophage colony-stimulating factors (M-CSF). The cells may also be incubated with additional immune stimulators such as LPS and/or interferon gamma (IFN-y). Use of such stimulators may induce different polarizations that mimic *in vivo* phenotypes (Mosser et al, 2008).

In certain embodiments, the phagocytic cells may comprise cargo proteins as described herein that do not have any effect on the phagocytic cells themselves but are secreted to affect the behavior of other cells.

In certain embodiments, the phagocytic cells of the invention are autologous. In other words, the phagocytic cells may be derived from a subject to be treated and thereby reduce the risk of immunological rejection.

Techniques are available in the art to generate phagocytic cells in large numbers for autologous cell-based therapies. Typically, phagocytic cells such as macrophages are produced from progenitor cells. For example, such progenitor cells may be established by overexpression of Hoxb8 in media supplemented with GM-CSF (Redecke et al., 2013). This results in rapidly proliferating, cloneable cells. Removal of Hoxb8 activity allows progenitors to differentiate into phagocytic cells (e.g. macrophages).

The invention also provides populations of more than about 10⁵, 10⁶, 10⁷ or 10⁸ phagocytic cells. In certain embodiments, the population comprises at least 10⁹, 10¹⁰, 10¹¹ or 10¹² phagocytic cells. The population is preferably homologous and/or autologous.

The phagocytic cells may be isolated, substantially isolated, purified or substantially purified. The phagocytic cells are isolated or purified if they are completely free of any other components, such as culture medium, other cells or other cell types. The phagocytic cells are substantially isolated if mixed with carriers or diluents, such as culture medium, which will not interfere with their intended use.

The phagocytic cells may be isolated using any suitable technique. For example, the phagocytic cells may be isolated by leukapheresis and/or elutriation (Faradji et al, 1994; Andreeson et al. 1990). Typically, the phagocytic cells are isolated from peripheral blood of a subject as described herein. The phagocytic cells (e.g. monocytes) may then be collected from blood and isolated using a combination of leukapheresis and elutriation.

As discussed in more detail below, the phagocytic cells are treated *ex vivo.* In particular, the phagocytic cells are loaded with polyhedrin protein crystals (themselves encapsulating one or more cargo proteins as described herein) and used therapeutically in the methods of the invention.

In certain embodiments, the phagocytic cells are provided in frozen aliquots and substances such as DMSO may be present to facilitate survival during freezing. Such frozen cells will typically be thawed and then placed in a buffer or medium either for maintenance or for administration.

In certain embodiments, the phagocytic cells are provided in a package. For example, the package may protect the cells from damage during collection, cell culture or transport. Suitable packages include, for example, Teflon bags or the like.

### Polyhedrin protein crystals

The phagocytic cells of the invention comprise one or more polyhedrin protein crystals (e.g. PODS^{®} protein crystals). Typically, the crystals are derived from viruses such as cypoviruses and baculoviruses. For example, the crystals may be derived from *Bombyx mori* cypovirus.

In certain embodiments, polyhedrin protein crystals are generated in cells in which the polyhedrin protein is expressed at high levels under the control of a promoter. Co-crystals are formed when a second protein is co-expressed and incorporated into the crystal. This second protein is termed the active, or cargo, protein. Typically, the promoters are polyhedrin promoters. Typically, the crystals are generated in insect cells. For example, the crystals may be generated using *Spodoptera frugiperda* 9 (sf9) cells.

In certain embodiments, the cargo protein is tagged with a short peptide sequence which causes the cargo protein to bind to the growing polyhedrin crystal. As the crystal continues to grow, the cargo protein becomes encased. As such, the crystals encapsulate one or more cargo proteins.

In certain embodiments, the polyhedrin protein comprises the *Bombyx mori* cypovirus polyhedrin sequence as set forth in SEQ ID NO: 1. Alternatively, the polyhedrin protein may comprise a sequence which has at least about 70%, 80%, 85%, 90%, 95%, 96%, 97%, 98%, 99% or more homology to the sequence of SEQ ID NO: 1 based on amino acid identify over the entire sequence of SEQ ID NO: 1.

In certain embodiments, the one or more cargo proteins comprise a polyhedrin binding tag selected from the H1-tag sequence set forth in SEQ ID NO:2. Alternatively, the polyhedrin binding tag may comprise a sequence which has at least about 70%, 80%, 85%, 90%, 95%, 96%, 97%, 98%, 99% or more homology to the sequence of SEQ ID NO: 2 based on amino acid identify over the entire sequence of SEQ ID NO: 2.

In certain embodiments, the one or more cargo proteins comprise a polyhedrin binding tag selected from the VP3-tag sequence set forth in SEQ ID NO:3. Alternatively, the polyhedrin binding tag may comprise a sequence which has at least about 70%, 80%, 85%, 90%, 95%, 96%, 97%, 98%, 99% or more homology to the sequence of SEQ ID NO: 3 based on amino acid identify over the entire sequence of SEQ ID NO: 3.

In certain embodiments, the one or more cargo proteins comprise a polyhedrin binding tag selected from the PH-tag sequence set forth in SEQ ID NO:4. Alternatively, the polyhedrin binding tag may comprise a sequence which has at least about 70%, 80%, 85%, 90%, 95%, 96%, 97%, 98%, 99% or more homology to the sequence of SEQ ID NO: 4 based on amino acid identify over the entire sequence of SEQ ID NO: 4.

In certain embodiments, the crystals are internalized within (e.g. inside) the phagocytic cell. For example, the crystals may be situated internally within a phagosome or phagolysosome or within the cytoplasm or nucleus of the cell rather than externally on the cell membrane.

In certain embodiments, intact crystals are capable of secreting the cargo proteins from within the phagocytic cell. In other words, a cargo protein may be released from the crystals and retain its natural conformation and/or function. Typically, the cargo proteins are released through the action of proteases (e.g. matrix metalloproteases) within the cell.

In certain embodiments, the cargo proteins have a desired phenotypic effect upon the phagocytic cell in which they are secreted. For example, cargo proteins may be capable of repolarizing the phagocytic cell (e.g. to a M1 macrophage phenotype).

In certain embodiments, the cargo proteins are released from the phagocytic cells over a sustained period of time. For example, the cargo proteins may be released from the cell across the cell membrane. Unexpectedly, the cargo proteins are capable of modifying the behavior of neighboring cells that may not themselves contain any crystals. In certain embodiments, the cargo proteins are released from the cell via exosomes. These exosomes may be harvested for therapeutic or research applications.

Typically, the cargo proteins are sustainably released over a period of time that is longer than that required for the phagocytic cell to reach and/or infiltrate its intended target. For example, the cargo proteins may be sustainably released from the phagocytic cells over a period of at least 2 days, 5 days, 7 days, 10 days, 14 days, 20 days, 21 days, one month or more.

Typically, the crystals are regular arrays of polyhedrin protein assembled in a crystal lattice. The crystals may be cubic or any other shape. The crystals of the invention are typically isolated from cells, such as insect cells, which have been infected with virus, typically baculovirus or cypovirus. The crystals are typically smaller than 10 microns, for example 0.1 to 6 microns (measured as the maximum distance inside the crystal between different surfaces or the maximum length of an edge). The crystals may be treated prior to use to modify their shape and/or reduce their size.

In certain embodiments, the crystals are isolated from the cells e.g. by centrifugation. Typically, the crystals are prepared in an aqueous suspension. For example, the concentration of the crystal suspension may be more than 10⁴ crystals per millilitre volume, typically from 10⁴ to 10⁷ crystals per millilitre volume, preferably from 5 × 10⁴ to 5 ×10⁶ or 10⁵ to 10⁶ crystals per millilitre volume. Crystals can be applied to (contacted with) the culture system/medium at these concentrations. These can be used in therapy at concentrations of 10⁵ to 10⁷ crystals per microliter, for example at 3×10⁶ crystals per millilitre.

Advantageously, the crystals are highly stable and rigid. The crystals may be attached (e.g. dried to a solid support). Typically, high levels of bioactivity are maintained after 6 months storage in protease-free solution at 37°C.

### Cargo proteins

The polyhedrin protein crystals as described herein encapsulate one or more cargo proteins, wherein the cargo protein comprises one or more therapeutic or detectable agents.

The phagocytic cells may comprise polyhedrin protein crystals comprising a single type of cargo protein. Alternatively, the phagocytic cells may comprise polyhedrin protein crystals comprising different types of cargo protein (e.g. a combination of cargo proteins). For example, the phagocytic cells may comprise at least 2, 3, 4, 5, 6, 7, 8, 9, 10 or more different cargo proteins.

As further described herein, the cargo proteins typically comprise a polyhedrin binding tag. The cargo proteins are typically secreted from the crystals intact. The cargo proteins are typically sustainably released from phagocytic cells. The cargo proteins may lead to a desired phenotypic effect within the phagocytic cell in which they are secreted and/or in neighboring cells.

In embodiments where there are more than one cargo proteins, each protein may have the same polyhedrin binding tag. Alternatively, each protein may have different polyhedrin binding tags.

In certain embodiments, the crystals are coated with an agent that delays the release of polyhedrin and cargo protein.

In certain embodiments, the cargo proteins comprise one or more therapeutic agents. For example, the cargo proteins may comprise one or more different antibodies, immunogens, enzymes, signaling proteins, hormones, growth factors and/or cytokines.

In certain embodiments, the one or more therapeutic agents are capable of re-programming M2 tumor-associated macrophages (TAMS), inflammatory monocytes and/or myeloid derived suppressor cells (MDSCs) into M1 macrophage cells. The ability of a therapeutic agent to reprogram M2 cell production can be determined, for example, by identifying and/or quantifying marker expression as described herein.

In certain embodiments, the one or more therapeutic agents activate STAT1, activate Notch, promote synthesis of ROS and/or NO release, promote AKt1 kinase activity, activate NF-κB signaling, or inhibit JNK activity or CSFR1.

In certain embodiments, the one or more therapeutic agents comprises one or more Th-1 related cytokines and/or TLR- ligands.

In certain embodiments, the one or more therapeutic agents are capable of stimulating M1 macrophage cell production. The ability of a therapeutic agent to stimulate M1 macrophage cell production can be determined, for example, by identifying and/or quantifying marker expression within a population of macrophages after being contacted with the therapeutic agent. As exemplified in Table 1, different subsets of macrophages (in humans) have distinct phenotypes.

**Table 1, adapted from Roszer et al. 2015 and Duluc et al. 2007**

| | **M1** | **M2a** | **M2b** | **M2c** | **M2d** |
|---|---|---|---|---|---|
| **Stimulation/activation** | IFN- γ | IL-4 | ICs | IL-10 | IL-6 |
| | LPS | IL-13 | IL-1R | TGF-beta | LIF |
| | GM-CSF | Fungal and Helminth infection | | GCs | Adenosine |
| **Marker expression** | CD86 | CD163 | CD86 | CD163 | VEGF |
| | CD80 | MHC II | MHC II | TLR1 | |
| | CD68 | SR | | TLR8 | |
| | MHC II | MMR/CD206 | | | |
| | IL-1R | CD200R | | | |
| | TLR2 | TGM2 | | | |
| | TLR4 | DecoyR | | | |
| | iNOS | IL-1R II | | | |
| | SOCS3 | *Mouse only:* | | | |
| | | *Ym1*/*2* | | | |
| | | *Fizz1* | | | |
| | | *Arg-1* | | | |
| | | | | | |

| **Cytokine secretion** | TNF | IL-10 | IL-1 | IL-10 | IL-10 |
|---|---|---|---|---|---|
| | IL-1 beta | TGF-beta | IL-6 | TGF-beta | IL-12 |
| | IL-6 | IL-1ra | IL-10 | | TNF-alpha |
| | IL-12 | | TNF-alpha | | TGF-beta |
| | IL-23 | | | | |
| **Chemokine secretion** | CCL10 | CCL17 | CCL1 | CCR2 | CCL5 |
| | CCL11 | CCL22 | | | CXCL10 |
| | CCL5 | CCL24 | | | CXCL16 |
| | CCL8 | | | | |
| | CCL9 | | | | |
| | CCL2 | | | | |
| | CCL3 | | | | |
| | CCL4 | | | | |

In certain embodiments, the one or more therapeutic agents are selected from any one or more proteins as recited in Table 1.

In certain embodiments, the one or more therapeutic agents are selected from one, two, three, four, five, six, seven, eight, nine, ten or more of the proteins recited in Table 1.

In certain embodiments, the one or more therapeutic agents are selected from any one or more of granulocyte-macrophage colony-stimulating factor (GM-CSF), CSF-2, CSF-1 (M-CSF), CSF-3 (G-CSF), interferon gamma (IFN-γ), TNF-α, IFN-β, IL-6, IL-12, IL-15, IL-15R, IL-21, IL-23, MHC-II, IL-1R, TLR2, TLR4, CD80, CD86, CD68, IL-1, IL-1 beta, IL-2, CCL2, CCL3, CCL4, CCL5, CCL8, CCL9, CCL10, CCL11, CXCL8, CXCL9, CXCL10, CXCL16, stem cell factor (SCF), histidine-rich glycoprotein, paclitaxel, SOCS3, Activin A, BtK, iNOS and/or NOS2.

In certain embodiments, the one or more therapeutic agents comprise IFN-γ, GM-CSF and/or TNFα. Typically, the one or more therapeutic agents comprise GM-CSF.

In certain embodiments, the cargo proteins comprise one or more detectable agents. For example, the cargo proteins may comprise a fluorescent and/or bioluminescent label, enzymatic label, chemiluminescent labeled or a biotinyl group. Radioisotopes or radionuclides may include ³H, ¹⁴C, ¹⁵N, ³⁵S, ⁹⁰Y, ⁹⁹Tc, ^{m}In, ¹²⁵1, ¹³¹I, fluorescent labels may include rhodamine, lanthanide phosphors or FITC and enzymatic labels may include horseradish peroxidase, β-galactosidase, luciferase, alkaline phosphatase.

Typically, the detectable agent is a fluorescent label such as GFP ("Green Fluorescent Protein"). For example, the detectable agent may be enhanced GFP (eGFP). Alternatively, the detectable agent may a bioluminescent label such as luciferase.

### Phagocytosis

The phagocytic cells of the invention may be harvested or produced by any suitable method.

In certain embodiments, the phagocytic cells are contacted with the polyhedrin protein crystals. Typically, phagocytic cells (e.g. monocytes) are contacted with the crystals. Such cells may then be unaffected by the crystal or differentiated or transdifferentiated (e.g. to M1 macrophages) after contact with the crystals.

In alternative embodiments, the crystals may be contacted with the phagocytic cells after they have been cultured and/or differentiated.

In certain embodiments, the crystals comprise cargo that is capable of stimulating macrophage (e.g. M1 macrophage) production (e.g. GM-CSF). In addition, or alternatively, the culture media may be supplemented with one or more agents that stimulate differentiation (e.g. LPS, IFN-γ, GM-CSF).

In certain embodiments, the crystals are attached to a solid support prior to contacting the phagocytic cells. Examples of suitable solid supports include, but are not limited to slides, membranes and tissue culture plates. Microcarriers may be also used. The crystals may be maintained in a non-dried form and, for example, centrifuged and/or dried to a support shortly before contact with the phagocytic cells.

In alternative embodiments, the crystals are pre-mixed with the phagocytic cells. Typically, the crystals are pre-mixed with phagocytic cells for up to 12, 24, 36, 48 hours or more.

The phagocytic cells may be cultured under any conditions which induce phagocytosis, including the methods further described herein. Phagocytosis of the crystals into the cells may be determined by any suitable immunohistochemistry (IHC) technique. In certain embodiments, the crystals themselves may comprise cargo (e.g. detectable agents such as GFP and/or luciferase) that allow phagocytosis into the cells to be verified (e.g. by fluorescent microscopy).

In certain embodiments, the phagocytic cells may be cultured in complete medium. Typically, the complete medium is supplemented with PMA. Typically, the phagocytic cells are cultured for up to 12, 24, 36, 48 hours or more.

As an example, the phagocytic cells may be cultured in complete medium (e.g. DMEM supplemented with 4.5g/L D-Glucose, L-glutamine, 10% heat-inactivated FBS, 100 U/mL penicillin and 100 µg/mL Glutamax optionally supplemented with 20 ng/mL M-CSF) at a seeding density of 0.5-1.0 e6/ml at 37ºC.

In certain embodiments, the phagocytic cells may be differentiated into polarizations that mimic *in vivo* phenotypes (e.g. a cytotoxic phenotype). For example, the culture media may be supplemented with additional immune stimulators such as LPS and/or IFN-γ to induce M1 macrophage production. Typically, 100 ng/ml LPS or 20 ng/ml IFN-γ is added to complete medium to induce M1 macrophage production after 12 hours culture in complete medium.

In certain embodiments, the phagocytic cells may be unaffected by the ingested crystals which contain a protein, such as IL-2, that targets other cells in diseased tissue.

In certain embodiments, the method further comprises formulating the one or more phagocytic cells using any suitable method including those further described herein. Typically, the one or more phagocytic cells are formulated for delivery via an injectable route.

### Pharmaceutical compositions

The invention provides a pharmaceutical composition comprising (a) a phagocytic cell of the invention or a population of phagocytic cells of the invention and (b) a pharmaceutically acceptable carrier or diluent. Typically, the phagocytic cells of the pharmaceutical composition comprise any one or more therapeutic agents as described herein.

The composition of the invention may be formulated using any suitable method. Formulation of cells with standard pharmaceutically acceptable carriers and/or excipients may be carried out using routine methods in the pharmaceutical art. The exact nature of a formulation will depend upon several factors including the cells to be administered and the desired route of administration. Suitable types of formulation are fully described in Remington's Pharmaceutical Sciences, 19th Edition, Mack Publishing Company, Eastern Pennsylvania, USA.

The composition of the invention may be administered by any route. Suitable routes include, but are not limited to, intravenous, intramuscular, intraperitoneal or other appropriate administration routes. Compositions may be prepared together with a physiologically acceptable carrier or diluent. Typically, such compositions are prepared as liquid suspensions of cells. The cells may be mixed with an excipient which is pharmaceutically acceptable and compatible with the active ingredient. Suitable excipients are, for example, water, saline, dextrose, glycerol, of the like and combinations thereof.

Typically, the composition is formulated for delivery via an injectable route.

In certain embodiments, the composition is used in combination with one or more other therapies intended to treat the same subject. By combination, it is meant that the therapies may be administered simultaneously, in a combined or separate form, to the subject. The therapies may be administered separately or sequentially to the subject as part of the same therapeutic regimen. For example, the composition of the invention may be used in combination with another therapy intended to treat cancer (e.g. solid cancer) in the subject.

In certain embodiments, treatment with one or more chemotherapeutic agents may be combined with the pharmaceutical composition of the invention. Typically, the chemotherapeutic agents are selected from Dacarbazine (DTIC), Temozolomide, Nab-paclitaxel, Paclitaxel, Carmustine (BCNU), Cisplatin, Carboplatin, Vinblastine, or any combination thereof.

In certain embodiments, treatment with one or more biological agents may be combined with the pharmaceutical composition of the invention. For example, the biological agent may be a growth factor/cytokine/chemokine such as IL-2. In certain embodiments, the biological agents are checkpoint inhibitors (e.g. anti-PD, PD-L1 and/or CTLA4 therapy). For example, the biological agent may be selected from ipilumab, nivolumab, atezolizumab, pembrolizumab and/or any combination thereof. For example, the biological agent may be selected from nivolumab and ipilimumab. In some embodiments, the biological agent is a B-Raf inhibitor, such as vemurafenib and/or dabrafenib. In certain embodiments, the biological agent is nivolumab and/or ipilimumab; dabrafenib and/or trametinib; vemurafenib and/or cobimetinib; and/or any combination thereof.

### Cell therapy

The invention further provides phagocytic cells, populations or pharmaceutical compositions as described herein for use in medicine. Therefore, the invention includes phagocytic cells as claimed herein for use in methods of preventing or treating any disease.

The invention provides a pharmaceutical composition comprising the phagocytic cells as claimed herein for use in a method of treatment of the human or animal body by therapy. For example, the therapeutic target may be any cell, extracellular vesicle and/or free protein in the tumor microenvironment. Herein, the term "tumor microenvironment is understood to include the environment around a solid cancer, including the surrounding blood vessels, immune cells, fibroblasts, signaling molecules and extracellular matrix (ECM).

In certain embodiments, the therapeutic target of the protein released by the macrophage is another type of immune cell, for example, a T cell or a dendritic cell. The target may also be a stromal cell or a cancer cell.

In certain embodiments, the invention provides a pharmaceutical composition for use in medicine, wherein an immunologically effective number of professional phagocytic cells of the invention, which are loaded with the polyhedrin protein crystals, are administered to a subject, thereby delivering the one or more therapeutic agents to a desired site. For example, the use of phagocytic cells allows the direct delivery of the one or more cargo proteins directly to a site of disease (e.g. cancer).

In certain embodiments, cells and/or tissue is remodeled at the site of the disease. For example, any cell, extracellular vesicle or free protein in the tumor microenvironment may be modulated as described herein. Typically, neighboring or adjacent phagocytic cells (e.g. macrophages) may be modulated at the site of the disease. Alternatively, the therapeutic target of the cargo protein may be another type of immune cell e.g., a T cell, dendritic cell, stromal cell, cancer cell or the like.

In certain embodiments, the invention concerns using the phagocytic cells to stimulate M1 macrophage cell production. In one embodiment, stimulating M1 macrophage production and/or reprogramming M2 associated TAMs, MDSCs, inflammatory M1 macrophages into M1 macrophage cells will help to remove a solid cancer (e.g. tumor) from the subject.

In certain embodiments, the disease is associated with M2 macrophage activation as further described herein. The skilled person would be able to determine whether a particular disease is associated with M2 macrophage activation. For example, the skilled person would be able to phenotype / score macrophages at the site of a disease using markers such as those described in Table 1.

In certain embodiments, the disease is a cancer. For example, the cancer may be associated with M2 macrophage activation and/or a solid cancer.

In certain embodiments, the phagocytic cells may be used to treat or prevent a solid cancer selected from breast, brain, bone, bone marrow, skin, colon, liver, colorectal, prostate, stomach, gastric, ovary, oral cavity, esophagus, pancreas, gallbladder, lung, thyroid, endometrium, head and neck, renal, bladder and/or glioma.

In certain embodiments, administration of the phagocytic cells acts to increase the proportion of M1 macrophages at the site of the disease. For example, the number of M1 macrophages may be increased by 1.1x, 1.2x, 1.3x, 1.4x, 1.5x, 2x, 3x, 10x, 15x, 20x, 100×, 1000× or more.

In certain embodiments, administration of the phagocytic cells acts to reduce the proportion of M2 macrophages at the site of the disease. For example, the number of M2 macrophages may be reduced by 1.1x, 1.2x, 1.3x, 1.4x, 1.5x, 2x, 3x, 10x, 15x, 20x, 100×, 1000× or more.

In certain embodiment administration of the phagocytic cells acts to reduce angiogenesis, invasion, metastasis, growth and/or cell proliferation of the disease.

The phagocytic cells of the invention may be administered to any suitable subject. For example, the subject may be murine, ovine, bovine, porcine, equine or any other mammal. The subject may be a human. The subject may be an infant, a juvenile or an adult. Typically, the subject is suffering from, susceptible to, or at risk from, the relevant disease. For instance, the subject may be suffering from or genetically predisposed to developing a cancer e.g. a solid cancer.

The phagocytic cells of the invention may be administered in a manner compatible with the dosage formulation and in such amount will be immunologically, prophylactically and/or therapeutically effective. The quantity to be administered may depend on the subject to be treated, capacity of the subject's immune system to respond to the antigen, and the degree of response desired.

Precise amounts of the phagocytic cells / pharmaceutical composition required to be administered may depend on the judgement of the practitioner and may be peculiar to each subject.

Any suitable number of cells may be administered to a subject. For example, at least, or about, 10⁵, 10⁶, 10⁷, 10⁸, 10⁹ or more cells may be administered. As a guide, the number of cells of the invention to be administered may be from 10⁵ to 10⁹, preferably from 10⁶ to 10⁸. Any of the specific numbers discussed above with reference to the populations of the invention may be administered. Typically, one or more doses of the phagocytic cells are administered to the subject.

In such cases where cells are administered or present, culture medium may be present to facilitate the survival of the cells. In some cases, the cells of the invention may be provided in frozen aliquots and substances such as DMSO may be present to facilitate survival during freezing. Such frozen cells will typically be thawed and then placed in a buffer or medium either for maintenance or for administration.

In certain embodiments, the invention provides the phagocytic cells as claimed herein for use in an autologous cell therapy.

In certain embodiments, one or more phagocytic cells are derived from peripheral blood as described herein. For example, the phagocytic cells may be derived from monocytes and cultured, propagated and/or differentiated using methods known in the art including those described herein.

In certain embodiments, the phagocytic cells are contacted with polyhedrin protein crystals under conditions which induce phagocytosis as described herein. For example, the crystals may be contacted with undifferentiated phagocytic cells (e.g. monocytes). Such cells may then be cultured and differentiated into macrophages (e.g. M1 macrophages) after contact with the crystals. Alternatively, the crystals may be contacted with the phagocytic cells after they have been cultured and/or differentiated.

In certain embodiments, the phagocytic cells are cultured and/or differentiated *ex vivo* using methods known in the art including those described herein.

In certain embodiments, the phagocytic cells are isolated and/or formulated with a pharmaceutically acceptable carrier or diluent using methods known in the art including those described herein.

In certain embodiments, the phagocytic cells as claimed herein are for use in an autologous therapy, wherein the cells are returned to the subject.

The skilled person will recognize that further modes of administration, dosages of therapeutic agents and treatment regimens can be determined by the treating physician according to methods known in the art.

### Imaging

The invention also provides a phagocytic cell for use in a method of imaging any solid cancer as described herein, wherein the phagocytic cell is a professional cell and comprises one or more polyhedrin protein crystals, wherein the crystals encapsulate one or more detectable agents.

In certain embodiments, the subject is murine or human as described herein.

In certain embodiments, the one or more detectable agents may be a fluorescent agent (e.g. GFP) or luminescent agent (e.g. luciferase).

The method further comprises detecting the detectable agent and thereby imaging at least part of the cancer. Any suitable method may be used to detect the detectable agent, including methods as further described herein.

### Examples

### Example 1 - Phagocytosis of PODS^{®} protein crystals into professional phagocytes

It was investigated whether PODS^{®} protein crystals can be phagocytosed by professional phagocytes such as monocytes, neutrophils and macrophages. PODS^{®} eGFP were adhered as a monolayer onto wells of a 12-well plate prior to seeding undifferentiated THP-1 cells (monocytes) on top of the crystals. In a different setup, PODS^{®} eGFP were briefly mixed with monocytes before plating the mixture onto wells in a homogeneous manner. After a 24 h incubation period, cells were stained and imaged. In either experimental configuration, multiple PODS^{®} proteins were successfully taken up by monocytes (Figure 1 a-d). In some cases, over 35 individual crystals were ingested by a single cell. Phagocytosis of the crystalline protein depots was independent of the embedded cargo protein as different PODS@ crystals containing either no protein (PODS^{®} Empty), growth factors, cytokines, chemokines or fluorescence proteins were all taken up efficiently.

THP-1 monocytes were differentiated into M0 macrophages by incubating cells in complete medium supplemented with PMA (Genin et al 2015, Del Arosa et al 2016). After incubating in differentiation medium for 24 hours, cells started to adhere and changed their morphology as well as their cytokine secretion pattern, e.g. increase of Activin A secretion, demonstrating differentiation into M0 macrophages. Subsequently, M0 macrophages were differentiated further into either M1 or M2 macrophages and PODS^{®} eGFP protein crystals were added to the each of the M0, M1 and M2 cell cultures. Cultures were further incubated and phagocytosis of PODS@ crystals were observed after 48 h (Figure 2 a-c). While M0 and M2 phagocytes exhibited similar levels of phagocytosis, with almost all PODS^{®} protein crystals ingested (Figure 2a and c, respectively), M1 macrophages were less efficient at ingesting the particles (Figure 2b). Our observations suggest that efficiency for ingesting PODS^{®} crystals as M2>M0>M1.

M0 macrophages were also assessed for their mobility and thus viability during and post phagocytosis. After the addition of PODS@ eGFP protein crystals, an M0 culture was monitored for 48 hours with a real-time cell history recorder in both brightfield and fluorescence channels at a rate of 1 frame/minute (Figure 3a -3b). Stills of the time-lapse recording demonstrate that many M0 macrophages were mobile and thus healthy during and after PODS^{®} eGFP were phagocytosed. Successful phagocytosis could be detected by observing a change in the position of the green fluorescent PODS@ from static (i.e. surface adhered) to mobile after being taken up into phagocytic cells. Furthermore, differentiated macrophages, i.e. M0, M1 and M2, typically are adherent when healthy and no detachment of cells was observed during the incubation period, even after many PODS^{®} protein crystals were taken up into each of the cells.

### Example 2 - Phagocytosis of PODS^{®} protein crystals into non-professional phagocytes

It was next determined whether the shape and size of PODS^{®} protein crystals would be equally conducive for phagocytosis into non-professional phagocytes, such as fibroblasts, osteochondrogenic cells, lymphocytes, erythrocytes, epithelial, myoblast or myocyte cells. Again, PODS^{®} eGFP were seeded as a dense, single-layer into wells of a 96 well plate and dried on. Subsequently, either fibroblast cells or chondrocyte cells were seeded on top of the adhered crystals and cultured for up to 48 h. As with macrophages, most of the seeded PODS^{®} protein crystals were taken up efficiently by both fibroblasts (Figure 4a) as well as chondrocytes (Figure 4b), aggregating within the cells, and very few protein crystals could be found in the space between cells. Similar to differentiated macrophages, fibroblasts and chondrocytes are adherent cells and would detach when unhealthy or apoptotic. However, following ingestions of PODS@ crystals, cells remained attached to the surface and continued to proliferate into an over-confluent state.

It was next determined whether the PODS^{®} had been ingested and not simply adhered to the surface of macrophages. Phagocytosis was demonstrated by incubating either undifferentiated THP-1 monocytes or M1 macrophages with PODS@ Empty (pure polyhedrin protein crystals, without cargo), for 24 h and then fixing cells such that their membranes were impermeable to antibodies. Cells were then subjected to an anti-polyhedrin primary antibody, which would only recognize PODS@ crystals that had not been phagocytosed and were attached to the cell surface or remained adhered to the TC plastic surface. Crystals recognized by the primary antibody were visualized using a FITC secondary antibody and a fluorescence microscope. When observing THP-1 monocytes using brightfield microscopy, almost all cells had ingested multiple PODS@ crystals (Figure 5a). After switching to the fluorescence channel, an estimated 90% of crystals had no equivalent fluorescent signal compared to the brightfield image. This provided clear evidence that the polyhedrin crystals were located within THP-1 cells as PODS@ were not recognized by the antibody pair. In the case of M1 macrophages, a different level of phagocytosis could be observed where many more protein crystals could be found outside the boundaries of cells. Consequently, these PODS@ crystals were readily recognized by the antibody pair and thus clearly visible in the fluorescence channel (Figure 5b). Lastly, phagocytosis into non-professional phagocytes could be demonstrated by first incubating chondrocytes with PODS^{®} eGFP for 24 h. Cells were subsequently fixed, cytoskeleton and nuclei stained and imaged using confocal microscopy. Scanning through focal layers revealed that cytoskeleton, nucleus and PODS@ eGFP crystals were all in the same focal plane (Figure 5c).

### Example 3 - PODS^{®} exhibit specific bioactivity after phagocytosis

In order to assess whether phagocytosed PODS@ growth factors PODS@ GF still exhibit the specific bioactivity of the embedded cargo, a proliferation assay was performed using PODS@ GM-CSF and PODS^{®} SCF on undifferentiated monocytes (THP-1) in two different configurations. In the first configuration, PODS@ GF were seeded into wells of a 24-well plate as a fairly uniform, single layer of protein crystals and adhered to the TC surface. Monocytes were then seeded on to the same surface (Figure 6a, I) and cultured for 4 days. In this configuration, PODS@ GF crystals are readily phagocytosed (Figure 6a, II). In contrast, in the second configuration, where PODS@ GF crystals are adhered to the porous membrane of TC inserts and monocytes then seeded into wells, PODS^{®} and cells are physically separated and thus phagocytosis is prevented (Figure 6a, III). Throughout the experiment, only cells are present on the surface of the well and are therefore unable to ingest protein crystals (Figure 6a, IV). After 4 days of culturing, final cell numbers were assessed using a colorimetric assay. Irrespective of whether PODS@ crystals were phagocytosed or not (Figure 6b, solid grey bars and dotted bars, respectively), each growth factor cargo showed proliferation stimulation activity as cell numbers in their presence increased significantly compared to measurements of non-supplemented cells (Figure 6b, horizontal line). This demonstrates that phagocytosed PODS@ protein crystals can exhibit specific bioactivity and thus macrophages could be used in a Trojan horse strategy to deliver protein depots for sustained release.

### Materials & Methods

### PODS@ crystal synthesis

All PODS^{®} proteins were synthesized as previous described (Nishishita et al. Biomaterials 2011;32:3555-3563; Matsumoto et al. Sci Rep 2012;2:935). All constructs were fused to the H1 incorporation tag (US8554493B2). Briefly, baculovirus (BV) DNA and transfer DNA was co-transfected into standard *Spodoptera frugiperda* 9 (Sf9) cells using TransIT^{®}-Insect (Mirus Bio). Resulting infective BV was harvested and plaque purification then performed to isolate a single recombinant BV. Isolated plaques were first screened and positive BV then harvested, expanded and finally used to infect large scale Sf9 cells cultures to produce PODS@ crystals. Subsequently, crystals were harvested and purified by lysing Sf9 cells using successive rounds of sonication and PBS washes. Finally, purified PODS@ were sterility tested and lyophilized prior to use in experiments. Although equivalence depends on context, 1.5×10⁴ PODS@ crystals is approximately functionally equivalent to 1 ng of many standard growth factors, cytokines or chemokines in terms of bioactivity (Matsumoto et al. 2015).

### Cell culture and differentiation

Monocyte suspension cells (THP-1, ATCC) were cultured undifferentiated in RPMI-1640 supplemented with 2 mM L-glutamine and 10% FBS (complete medium). For M0 differentiation, THP-1 were centrifuged and the conditioned media replaced with fresh complete medium supplemented with 100 ng/ml phorbol 12-myristate-13-acetate (PMA, Sigma P8139). Cells were seeded and cultured for up to 12 days, with media changes every 3-4 days. After 24-48 h, M0 macrophages were further differentiated into M1 or M2 macrophages as described [Genin et al 2015, Del Arosa et al 2016]. Briefly, M0 differentiation medium was removed and adherent M0 cells washed twice with serum-free medium. For further differentiation, complete medium supplemented with either 100 ng/ml LPS and 20 ng/ml IFN-γ (M1) or 20 ng/ml IL-4 and 20 ng/ml IL-13 (M2) was added to adherent M0 cells and then incubated for up to a further 10 days.

### Phagocytosis

PODS@ crystals were either centrifuged onto tissue culture (TC) plates; or centrifuged and dried onto TC plates; or pre-mixed with phagocytic cells for up to 48 hours before plating. Phagocytic cells were then seeded where necessary and subsequently cultured in complete medium. Phagocytosis into cells was demonstrated either by monitoring with a real-time cell history recorder (JuLi stage, NanoEnTek Inc.) or by commonly used immunohistochemistry (IHC) stains and techniques.

To stain for polyhedrin protein specifically, cells were fixed with 10% formalin for 10 minutes and then blocked in 1% PBS-BSA for 1 hour. Subsequently, proprietary rabbit anti-polyhedrin (diluted 1:1000 in 1% PBS-BSA) was incubated on the cells for 1 h, followed by a 1% PBS-BSA wash three times. Lastly, anti-rabbit FITC secondary antibody (diluted 1:1000 in 1% PBS-BSA) was incubated for 1 h and washed with 1% PBS-BSA three times before mounting in ProLong Diamond Antifade mountant (ThermoFisher Scientific).

### Proliferation assay

PODS@ GM-CSF or PODS^{®} SCF (1.8x 10⁶ each) were seeded either directly onto the well of a 24-well plate as a single crystal layer or into 24-well TC inserts (1 µm pore size, Corning Falcon). Subsequently, 4000 monocytes were seeded into the wells that contained either a monolayer of PODS^{®} protein crystals; or an insert with PODS^{®} protein crystals; or an empty insert as baseline control. Cultures were then incubated for 4 days and final cell numbers assessed using a colorimetric assay (OrangU, Cell Guidance Systems).

### Discussion

The potential utility of self-assembling proteins in phagocytic cell-mediated strategies to treat disease is demonstrated herein. This utility has been demonstrated with polyhedrin crystals generated using the PODS^{®} expression system based on the polyhedrin protein derived from *Bombyx mori* cypovirus. Advantageously, it is demonstrated herein that:
(1) PODS@ crystals are taken up efficiently by both professional and non-professional phagocytic cells;
(2) Cells remain healthy following ingestion of PODS@ crystals and the polyhedrin protein alone has no detectable effect on the phenotype of the PCs;
(3) When the PODS@ crystals contain mitogenic cytokines, the proliferation rates of the phagocytic cells increase significantly showing that the cargo protein is released within the cell intact and is able to interact with its receptor;
(4) Cargo protein is secreted by PODS@ crystals;
(5) Co-cultured cells that have not ingested PODS^{®} crystals themselves respond to the cargo protein that has been secreted by the phagocytic cells which have ingested PODS^{®} crystals, allowing the phenotype of co-cultured cells to be altered.

Phagocytic cells are intimately associated with disease aetiology and tissue remodeling. The ability of macrophages, in particular, to infiltrate diseased tissues make them attractive targets for therapeutics and also as agents to deliver drugs.

Significant advances in the understanding of the role of the immune system and phagocytic cells in disease genesis has led to the development of effective new therapies. Proteins, including cytokines, have demonstrated therapeutic potential and many have received marketing approval, mainly for applications where repeated dosing is possible via systemic delivery. With some notable exceptions, such as IL-2 to treat melanoma and renal cell cancer, albeit with significant toxicity to non-target cells, the fragility of proteins has confounded attempts to use them either directly or in a Trojan horse strategy to treat disease.

Given the importance of phagocytic cells in disease and the therapeutic potential of cytokines and other fragile proteins, drug delivery devices that are able to sustain an effect specifically on phagocytic cells provide a platform technology for protein-based treatment strategies.

PODS@ crystals demonstrate extraordinary stability. For example, high levels of bioactivity are maintained after 6 months storage in protease-free solution at 37°C. In addition, PODS@ crystals have optimal size range (0.2-6 microns), shape (cubic) and elasticity (rigid). It is shown herein that PODS@ crystals are efficiently taken up by a wide range of professional and non-professional phagocytic cells.

Another remarkable feature of PODS@ crystals is their dynamic release mechanism which requires proteases. It has previously been shown that many matrix metalloproteases can mediate this release. It is shown herein, unexpectedly, that once taken up by phagocytic cells, PODS@ crystals persist for several weeks, the ingesting phagocytic cells remain healthy and that the cytokines released by PODS@ crystals continue to have a proliferative effect on the cell population. Moreover, it is demonstrated herein that cytokines are released from phagocytic cells and that the released cytokines are able to modulate the phenotype of adjacent cells that have not taken up the macrophages.

### Example 4 - PODS^{®} "Trojan horse" approaches

PODS@ are micron scale cubic co-crystals which contain stabilized recombinant protein cargo which is sustainably released under the activity of proteases. PODS^{®} are an ideal size, shape and stiffness for efficient phagocytosis. However, in order for a PODS^{®} Trojan horse approach and to be viable as a delivery tool for therapeutic proteins, several criteria have to be met. These include:
(1) Monocytes and macrophages need to tolerate phagocytosis of the micron-sized PODS^{®} without dramatic change to their viability and metabolism;
(2) Phagocytosis of micron-sized PODS@ should not alter macrophage's ability and characteristics of movement, including chemo-sensing and traversing small blood vessels and capillaries;
(3) Intracellular degradation of phagocytosed PODS@ protein crystals must result in release of intact and bioactive cargo protein; and
(4) PODS^{®}-filled phagocytic cells that release cargo protein should be able to influence the behavior of neighboring cells.

It is demonstrated herein that cubic PODS@ crystals, which are rigid micron-sized protein crystals, can be phagocytosed efficiently into professional and non-professional phagocytes. Furthermore, growth factors secreted from phagocytosed PODS@ may still elicit their specific biological activity such as proliferation of the phagocytic cells which has taken them up.

It is further demonstrated herein that, against expectations, PODS@ crystals satisfy all the criteria listed above which are required for therapeutic strategies using phagocytic cells as Trojan horses by either modulating the function of the macrophages themselves or orchestrating the behavior of other cells.

### Results

Given the cytotoxic effect generated by different types of nanoparticles on macrophages in previous studies (Lanone et al 2009, Akter et al 2018, Feng et al 2018), the viability of M0, M1 and M2 was examined after uptake of PODS@ crystals. For this experiment, THP-1 monocytes were differentiated into M0 macrophages by incubating cells in complete medium supplemented with PMA. M0 cells were then polarized to M1 and M2 type macrophages by incubating cells in complete medium supplemented with either IFN-γ and LPS or IL-4 and IL-13, respectively. Subsequently, cells were incubated for 24 h with different numbers of PODS^{®} Empty (containing no cargo protein) or PODS^{®} FGF-10 and tested for their viability 48 h and 96 h after uptake (Figure a and b). Results in WST-8 assays demonstrated that cell viability was not reduced compared with untreated cells, suggesting that the PODS^{®} particles were non-toxic at a median dosage range of up to 15 PODS/cell.

It was next examined if the movement of macrophages initiated by chemotactic signals is modulated by the phagocytosis of PODS. In order to quantify potential changes due to uptake of PODS@ into macrophages, M0 cells were incubated with PODS^{®} IL-2 for 24 h and then observed with a live cell imaging system under normal growth conditions for a further 24 h. Subsequently, movement of unloaded- and PODS^{®} IL-2-loaded cells was analyzed by tracking each of 25 cells across 720 frames; first using the manual tracker function of Image J and then using Ibidi's chemotaxis and migration tool (Figure a and b, respectively). When comparing tracks for both the distance migrated and randomness of mobility, there was no detectable difference in the mobility of cells with and without PODS^{®}. To test the ability of PODS^{®} IL-2-loaded M0 cells to follow a chemotactic gradient, the experiment was repeated with a slight variation: after PODS@ uptake, cells were taken into serum-free medium and transferred into chemotactic slides with two reservoirs. Reservoir 1 was filled with medium containing 10% serum, acting as a chemoattractant, and reservoir 2 was filled with serum free medium. The movement of unloaded- and PODS^{®} IL-2-loaded cells was investigated by tracking 26 cells each as described above **(Error! Reference source not found.**a and b, respectively). An analysis of this tracking data revealed that uptake of PODS@ crystals does not impair directed migration of macrophages to a chemoattractant source.

In order to assess if phagocytosis of PODS@ crystals is similarly efficient, compared to THP-1 cells, into primary cells, monocytes and macrophages were isolated from the tibias of C57BL/6 mice and cultured for 5 d. The cells were then incubated with PODS^{®} eGFP for 24 h and subsequently monitored with a live cell imaging system for a further 24 h. The murine primary cells were more mobile compared to the human monocytic cell line. They phagocytosed the PODS@ crystals with equally high efficiency and were also capable of ingesting similar numbers of PODS crystals. Figure 10 shows four frames in which a loaded cell (dashed circle) can be seen to stretch forward to collect and ingest another PODS@ crystal to its left.

Having demonstrated that PODS^{®}-loaded macrophages are still able to react to and follow chemotactic signals, it was assessed whether loaded cells retain their ability to traverse narrow spaces, another important feature of macrophages. M0 cells were incubated with PODS^{®} eGFP for 24 h, then taken into serum-free medium and transferred to 24-well tissue culture inserts containing 8 µm pores, a diameter which is akin to that of small blood vessels. The regular maintenance medium containing 10% serum was used as a chemoattractant in the bottom well (Figure 11b). A second well, but without any serum as chemoattractant, served as a control to show that cells in the bottom well have actively migrated down and not randomly traversed the pores or simply settled under gravity (Figure 11a). After a further 24 h incubation, both experimental chambers were analysed with brightfield and fluorescence microscopy. A large difference in cell numbers in the wells with and without chemoattractant demonstrated that, despite their PODS@ cargo, loaded M0 cells not only were capable of passing through the narrow pores but actively migrated through the 8 µm pores to the chemotactic source.

Importantly, macrophages are actively recruited by chemical signals from solid tumours (Dandenkar et al 2011), so it was tested if conditioned medium of a cancer cell line can attract PODS@ eGFP loaded macrophages using the same cell chamber as described above. A375 cells, a human malignant melanoma cell line, were grown for 3 days in 10% serum containing growth medium. The conditioned medium was then used as chemoattractant in the bottom well (Figure 12b). In a second cell chamber non-conditioned 10% serum containing medium in the bottom well served as a control to determine the level of non-directional macrophage movement in the absence of factors secreted by A375 cells (Figure 12a). Subsequent microscopy showed that PODS@ eGFP-loaded M0 cells were attracted specifically by the conditioned medium of the cancer cell line, engaging in directed migration through the 8 µm pores towards the chemotactic source.

The activation and polarization of monocytes towards an M1-like phenotype upon contact with foreign material is a key element of the innate immune system. To test if contact with and uptake of PODS@ crystals by monocytes and macrophages influences their polarization status, the secretion of IL-6 was tested, a marker for M1 polarization, before and after PODS@ uptake (Figure 13). First, it was confirmed that IL-6 can indeed be used as a proxy for polarization status, showing that M1 cells raised concentration levels of IL-6 in the cell culture media to more than 4 ng/ml directly after polarization, whereas neither non-activated THP-1 cells nor M0 and M1 cells raised IL-6 concentration above background level (Figure a). The polarization medium was then changed to medium containing PODS^{®} FGF-2 and phagocytic uptake was allowed to proceed for 24 h. Within this incubation time, both loaded and unloaded M1 cells raised IL-6 concentrations to 200 pg/ml. Unchanged levels of the polarization marker IL-6 between M1 cells which have taken up PODS@ crystals and M1 cells which have not been incubated with PODS@ crystals suggest that the uptake of PODS@ crystals did not alter the polarization status of M1 cells. Equally, the uptake of PODS@ crystals did not induce IL-6 secretion of M0 and M2 cells, suggesting that phagocytosis of PODS@ crystals does not change the polarization status of any macrophage type.

To test if cargo protein of ingested PODS^{®} is released from the macrophages into the medium, M0 cells were incubated with PODS^{®} IL-6 for 24 h, washed twice and then incubated for a further 4 days in normal growth medium containing no additional PODS. Then, medium was collected and analyzed for the presence of IL-6 by ELISA (Figure a). Surprisingly, not only was the cargo protein IL-6 detected in the cell culture medium but levels of IL-6 were dose-dependent: the more PODS^{®} IL-6 were loaded into M0 cells, the more could be detected in the medium after 4 days. As a comparison, the amount of IL-6 released from similar numbers of non-phagocytosed (naked) PODS^{®} IL-6 (Figure b) was also measured. The amount of IL-6 released from naked PODS@ was up to 10 times higher than from PODS@ taken up by macrophages. As a further control, M0 cells were incubated with PODS^{®} FGF-2 in the same way and levels of IL-6 in the medium were analyzed. Not only were IL-6 levels below background (<10 pg/ml) but also their levels did not change in a dose-dependent manner.

To investigate if PODS^{®} cargo protein released by macrophages is bioactive, a proliferation assay using NIH-3T3 cells that are responsive to FGF-2 protein was performed. NIH-3T3 cells were seeded in normal growth medium in a 24-well plate and after 24 h of growth, the media was replaced with serum-free conditioned medium. Macrophages were incubated with PODS^{®} FGF-2 (10 PODS^{®}/cell) for 24 h, taken into NIH-3T3 serum-free conditioned medium and seeded into 24-well TC inserts. Then, cells were co-incubated for a further 4 days and the number of NIH-3T3 cells assessed by performing a WST-8 assay (Figure). Co-incubation of all types of PODS^{®} FGF-2 loaded macrophages supported the growth of FGF-2 responsive NIH-3T3 cells in serum-free condition compared to empty macrophages (blue bars) and cells only (horizontal green line).

### Discussion

The potential utility of packaged proteins delivered by macrophages as a novel therapeutic drug delivery strategy has been explored. In particular, the utility of polyhedrin crystals generated using the PODS^{®} expression system based on the polyhedrin protein derived from Bombyx mori cypovirus has been investigated.

Selective targeting of drugs to diseased tissue, notably cancer, is needed to reduce levels of off-target toxicity and to increase efficacy. The use of cells as a therapeutic delivery system, also described as a Trojan horse strategy, has been under discussion since the late 1970s. Developing an effective particle which contains the drug, allows function of the macrophage and subsequent release has been challenging, particularly for protein drugs such as cytokines. Non-phagocytic cells also have potential for Trojan horse delivery. But here also, despite ongoing research, there is, to date, no cell-based delivery system approved for the clinic, although there are at least two therapeutics using red blood cells as drug vehicle are currently in phase III trials (NCT02770807, NCT03665441). The distinct advantage of macrophages over other cell delivery systems is their inherent disease-homing ability. As first line defenders in inflammation and as part of the foreign body response, they are directly attracted to the side of solid tumors and other diseases.

It is demonstrated herein that phagocytosed PODS@ proteins are non-toxic to multiple types of macrophages, even at high particle numbers. Furthermore, directed migration of macrophages that contain PODS@ crystals towards a chemoattractant is not impaired. Critically, loaded macrophages are still able to change their cell morphology to travel through narrow space as demonstrated by their ability to actively pass through pores of 8 µm diameter. Following phagocytosis, release is shown from macrophages of protein that was encapsulated into PODS@ crystals. Released cargo protein could be detected reliably and in a dose-dependent manner using ELISA in macrophage culture medium which had been conditioned with PODS-containing macrophages from as few as 5 PODS@ per macrophage. Even more surprisingly, released protein was shown to be bioactive as verified by proliferation of an FGF-2 reactive cell line upon co-incubation with PODS^{®} FGF-2 loaded macrophages. Thus, a PODS-based macrophage mediated Trojan horse strategy is developed for delivering drugs to cancer and other diseases.

### Material & Methods

### PODS^{®} crystal synthesis

All PODS^{®} proteins were synthesised as previous described (Nishihita et al 2011, Matsumo et al 2012). All constructs were fused to the H1 incorporation tag (Metcalf et al 2008). Briefly, baculovirus (BV) DNA and transfer DNA was co-transfected into standard Spodoptera frugiperda 9 (Sf9) cells using TransIT^{®}-Insect (Mirus Bio). Resulting infective BV was harvested and a plaque purification then performed to isolate a single recombinant BV. Isolated plaques were first screened and positive BV then harvested, expanded and finally used to infect large scale Sf9 cells cultures to produce PODS@ crystals. Subsequently, crystals were harvested and purified by lysing Sf9 cells using successive rounds of sonication and PBS washes. Finally, purified PODS@ were sterility tested and lyophilised prior to use in experiments. Although equivalence depends on context, 1.5×10⁴ PODS@ crystals is approximately functionally equivalent to 1 ng of many standard growth factors, cytokines or chemokines in terms of bioactivity (Matsumoto et al 2015).

### Cell culture and differentiation

Monocyte suspension cells (THP-1, Public Health England Culture Collection) were cultured undifferentiated in RPMI-1640 (A10491, Gibco^{®}) supplemented with 10% BCS (30-2030, ATCC) (complete medium). For M0 differentiation, THP-1 cells were centrifuged and the conditioned media replaced with fresh complete medium supplemented with 100 ng/ml phorbol 12-myristate-13-acetate (PMA, Sigma P8139). After 48 h, M0 macrophages were further differentiated into M1 or M2 macrophages as described (Rios de la Rosa et al 2017, Genin et al, 2015). Briefly, M0 differentiation medium was removed and adherent M0 cells were washed twice with serum-free medium. For further differentiation, complete medium supplemented with either 100 ng/ml LPS and 20 ng/ml IFN-γ (M1) or 20 ng/ml IL-4 and 20 ng/ml IL-13 (M2) was added to adherent M0 cells and then incubated for up to a further 48h. A375 cells (ATCC) were cultured in DMEM (41966, Gibco^{®}) supplemented with 10% FBS (F7524, SIGMA). Cells were seeded at a density of 2e4 cell/cm² and passaged every 3 days. TF-1 cells (ATCC) were cultured in RPMI-1640 supplemented with 2 ng/ml GM-CSF and 10% FBS. Cells were seeded at a density of 2e5 cells/ml and passaged twice a week.

### Phagocytosis

PODS^{®} crystals were either centrifuged onto tissue culture (TC) plates; or centrifuged and dried onto TC plates; or pre-mixed with phagocytic cells for up to 48 hours before plating. Phagocytic cells were then seeded where necessary and subsequently cultured in complete medium. Phagocytosis into cells was demonstrated either by monitoring with a real-time cell history recorder (JuLi stage, NanoEnTek Inc.).

### Viability assay

To test macrophage viability after PODS@ uptake, THP-1 cells were seeded at a density of 2e5 cells/ml and differentiated and polarized as described above. 1e6 or 3e6 PODS^{®} Empty and PODS^{®} FGF-10 per ml were added to unpolarized M0 and polarized M1 and M2 macrophages, resulting in an average of 5 or 15 PODS@/ cell, respectively. Cells were then incubated for 48h and 96h hours and a WST-8 assay (Orangu^{™}, Cell Guidance Systems) was performed to determine cell viability.

### Mobility tracking

THP-1 cells were seeded at a density of 2e5 cells/ml in 24-well plates and differentiated into M0 macrophages as described above. M0 cells were then incubated with PODS^{®} IL-2 for 24h in complete medium. The medium was then renewed and cells recorded using a real-time cell history recorder (JuLi stage, NanoEnTek Inc.). Images were taken every 2 min for 24 h and then analysed using the manual tracker of Image J and the chemotaxis and migration tool (Ibidi).

### Chemotaxis

THP-1 cells were seeded at a density of 2e5 cells/ml in T25 TC flasks and differentiated into M0 macrophages as described above. M0 cells were then incubated with PODS^{®} IL-2 for 24h in complete medium. Subsequently, M0 macrophages were washed with PBS and then detached using an enzyme-free detachment buffer (Cell Dissociation Buffer, Gibco^{®}). Cells were then centrifuged, re-counted and seeded in serum-free RPMI-1640 medium at a density of 2e6 cells/ml into Chemotaxis µ-Slides (Ibidi). 10% FBS was used as a chemoattractant. Cells in the observation area were recorded using a real-time cell history recorder (JuLi stage, NanoEnTek Inc.). Images were taken once per minute for 12 h and then analysed using the manual tracker of Image J, and the chemotaxis and migration tool (Ibidi).

### Directed migration

THP-1 cells were seeded at a density of 2e5 cells/ml in T25 TC flasks and differentiated into M0 macrophages as described above. M0 cells were then incubated with 5e5 PODS^{®} eGFP per mL for 24h in complete medium (2.5^{®} PODS/cell). Subsequently, M0 macrophages were washed with PBS and then detached using an enzyme free detachment buffer (Cell Dissociation Buffer, Gibco^{®}). Cells were centrifuged, re-counted and seeded in serum-free or 10% FBS containing RPMI-1640 medium at a density of 1e6 cells/ml into 24-well inserts. The bottom well was then filled with either serum-free medium, 10% FBS-containing medium or with 3 day conditioned medium of A375 cells. Microscope images of the bottom well and the insert were taken after 24h of migration.

### Endogenous IL-6 secretion

THP-1 cells were seeded at a density of 2e5 cells/ml in 24-well plates and differentiated and polarized as described above. The conditioned polarization medium was then collected and stored at -20 °C for later analysis. Macrophages were then incubated with 2e6 PODS^{®} FGF-2 per ml for 24h in complete medium (10 PODS^{®}/cell). The medium was then collected and stored at -20 °C. Medium samples were tested for the presence of IL-6 by ELISA (DY206, R&D systems) according to the manufacturer's protocol.

### Release of IL-6 from PODS@ IL-6 loaded macrophages

THP-1 cells were seeded at a density of 2e5 cells/ml in 96-well plates and differentiated into M0 macrophages. M0 cells were then incubated with 1e6, 2e6 or 3e6 PODS^{®} IL-6 per ml for 24h in complete medium (resulting in 5, 10 or 30 PODS^{®}/cell). Cells were then washed twice with PBS and fresh complete medium was added and cells were incubated for 4d. Additionally, the same number of naked PODS^{®} IL-6 were added to the wells of a 96-well plate and were spun down at 3000g for 25 min. The PBS was removed and the plate dried in a laminar flow hood and then supplemented with full growth medium for 4d. After incubation, the medium was collected and subsequently tested by IL-6 ELISA (DY206, R&D systems) according to the manufacturer's protocol.

### Functional assay

THP-1 cells were seeded at a density of 2e5 cells/ml in 6-well plates and differentiated and polarized as described above. Macrophages were then incubated with 2e6 PODS^{®} FGF-2 per ml for 24h in complete medium (10 PODS^{®}/cell). Subsequently, macrophages were washed with PBS and then detached using an enzyme-free detachment buffer (Cell Dissociation Buffer, Gibco^{®}). Cells were then centrifuged, re-counted and seeded in serum free TF-1 conditioned medium at a density of 2e6 cells/ml into 24-well inserts. TF-1 cells were seeded at a density of 3e4 cells/cm² in full growth medium into a 24-well plate. After one day of growth, medium was changed to RPMI-1640 supplemented with 0.5% BCS and the macrophage containing inserts were added. Cells were co-incubated for 4d and the viability of the TF-1 cells was measured performing a WST-8 assay (Orangu^{™}, Cell Guidance Systems).

### Derivation of primary monocytes from mouse tibia

Murine bone marrow derived monocytes were isolated according to Wagner et al. 2014. Briefly, the tibias of 3 C57BL/6 mice were prepared for harvest. After washing once in 96% ethanol and twice in PBS, the distal end of each bone was cut with fine scissors and the bones flushed with warm medium (M199 supplemented with 10% FBS and 1% Penicillin/Streptomycin) using a 28-G needle and a 1 ml syringe. The flow-through from the bones was collected and filtered through a 70 µm cell strainer. The cell suspension was centrifuged at 200 × g for 10 min at RT. The pellet was washed with 25 ml of medium and centrifuged again. The cells were then seeded at 0.5e6 cells/ml in 6-well ultra-low-attachment plates. Cells were cultured for 5 days before using in experiments.

Taken together, these results demonstrate the utility of self-assembling proteins and PODS@ crystals in particular to modulate the phenotype of phagocytic cells and adjacent cells. These findings have enabled the development of effective therapies for disease treatment and tissue remodeling as described herein.

### References

- Andersen et al. Long-Lasting Complete Responses in Patients with Metastatic Melanoma after Adoptive Cell Therapy with Tumor-Infiltrating Lymphocytes and an Attenuated IL2 Regimen (2016) DOI: 10.1158/1078-0432.CCR-15-1879.
- Andreeson et al. Cancer Res. 1990;50:7450-7456.
- Choi et al. (2007) A Cellular Trojan Horse for Delivery of Therapeutic Nanoparticles into Tumors Nanotechnology letters (12):3759-65.
- van Dalen et al. (2019) Molecular repolarization of tumor-associated macrophages Molecules 2019, 24, 9 DOI 10.3390.
- Faradji et al, Journal of immunological methods. 1994;174:297-309.
- Jiang et al. (2016) Role of IL-2 in cancer immunotherapy. Oncoimmunology. 5(6): e1163462.
- Matsumoto et al. J Biomater Appl 2015;30:193-200.
- Mosser et al, Current protocols in Immunology. 2008 14:14:12.
- Redecke et al., Nature Methods. 2013; 10: 795-803.
- Zhang et al, Current protocols in Immunology. 2008 14:14: 11.
- Xu, F. et al. Membrane-wrapped nanoparticles probe divergent roles of GM3 and phosphatidylserine in lipid-mediated viral entry pathways. Proc. Natl. Acad. Sci. 115, E9041-E9050 (2018).
- Pang, L. et al. A novel strategy to achieve effective drug delivery: exploit cells as carrier combined with nanoparticles. Drug Deliv. 24, 83-91 (2017).
- Rios de la Rosa, J. M., Tirella, A., Gennari, A., Stratford, I. J. & Tirelli, N. The CD44-Mediated Uptake of Hyaluronic Acid-Based Carriers in Macrophages. Adv. Healthc. Mater. 6, (2017).
- Nishishita, N. et al. The use of leukemia inhibitory factor immobilized on virus-derived polyhedra to support the proliferation of mouse embryonic and induced pluripotent stem cells. Biomaterials 32, 3555-3563 (2011).
- Matsumoto, G. et al. Bone regeneration by polyhedral microcrystals from silkworm virus. Sci. Rep. 2, 935 (2012).
- Lanone, S. et al. Comparative toxicity of 24 manufactured nanoparticles in human alveolar epithelial and macrophage cell lines. Part. Fibre Toxicol. 6, 14 (2009).
- Akter, M. et al. A systematic review on silver nanoparticles-induced cytotoxicity: Physicochemical properties and perspectives. J. Adv. Res. 9, 1-16 (2018).
- Feng, Q. et al. Uptake, distribution, clearance, and toxicity of iron oxide nanoparticles with different sizes and coatings. Sci. Rep. 8, 2082 (2018).
- Dandekar, R. C., Kingaonkar, A. V. & Dhabekar, G. S. Role of macrophages in malignancy. Ann. Maxillofac. Surg. 1, 150-154 (2011).
- Metcalf, P. et al. Viral polyhedra complexes and methods of use. (2008).
- Genin, M., Clement, F., Fattaccioli, A., Raes, M. & Michiels, C. M1 and M2 macrophages derived from THP-1 cells differentially modulate the response of cancer cells to etoposide. BMC Cancer 15, 577 (2015).
- Wagner, M. et al. Isolation and intravenous injection of murine bone marrow derived monocytes. J. Vis. Exp. JoVE (2014) doi:10.3791/52347.

## Claims

1. A phagocytic cell comprising one or more polyhedrin protein crystals, wherein the crystals encapsulate one or more cargo proteins, wherein the phagocytic cell is a professional cell and the cargo protein comprises one or more therapeutic agents.

2. The phagocytic cell according to claim 1, wherein:
(a) the crystals are internalized within the phagocytic cell;
(b) the crystals are capable of secreting the cargo proteins within the phagocytic cell intact; and/or
(c) the cargo proteins are capable of being sustainably released from the phagocytic cell,
wherein optionally the cells are mammalian cells, wherein further optionally the mammalian cells are human cells.

3. The phagocytic cell according to claim 1 or 2, wherein the crystals are smaller than 10 microns and/or substantially cubic in shape, wherein optionally:
(a) the polyhedrin protein comprises the Bombyx mori cypovirus polyhedrin sequence as set forth in SEQ ID NO:1; or
(b) the polyhedrin protein comprises a sequence which has at least 70% homology to the sequence of SEQ ID NO: 1 based on amino acid identity over the entire sequence of SEQ ID NO: 1,
wherein further optionally the cargo proteins comprise one or more polyhedrin-binding tag sequences selected from:
(a) the H1-tag sequence as set forth in SEQ ID NO: 2;
(b) the VP3-tag sequence as set forth in SEQ ID NO: 3;
(c) the PH tag sequence as set forth in SEQ ID NO: 4; or
(d) a sequence which has at least 70% homology to the sequence of SEQ ID NO: 2, 3 or 4 based on amino acid identity over the entire sequence of SEQ ID NO: 2, 3 or 4 respectively.

4. The phagocytic cell according to any one of claims 1 to 3, wherein the professional cell is a monocyte, macrophage or glial cell, wherein optionally the phagocytic cell is a macrophage.

5. The phagocytic cell according to any one of claims 1 to 4, wherein the phagocytic cell is derived from peripheral blood of a subject.

6. The phagocytic cell according to any one of claims 1 to 5, wherein the one or more therapeutic agents are capable of targeting a cell, extracellular vesicle or free protein in a tumor microenvironment.

7. The phagocytic cell according to claim 6, wherein:
(a) the one or more therapeutic agents are capable of stimulating M1 macrophage cell production; and/or
(b) the one or more therapeutic agents are capable of re-programming M2 tumor - associated macrophages (TAMS), inflammatory monocytes and/or myeloid derived suppressor cells (MDSCs) into M1 macrophage cells,
wherein optionally the one or more therapeutic agents are growth factors, cytokines, enzymes, signaling molecules, hormones, antibodies and/or vaccines, wherein further optionally the one or more therapeutic agents are selected from:
(a) Th-1 related cytokines and/or TLR-ligands; and/or
(b) granulocyte-macrophage colony-stimulating factor (GM-CSF), CSF-2, CSF-1 (M- CSF), CSF-3 (G-CSF), interferon gamma (IFN-γ), TNF-α, IFN-β, IL-6, IL-12, IL-15, IL-21 , IL-23, MHC-II, IL-1 R, TLR2, TLR4, CD80, CD86, CD68, IL-1 , IL-1 beta, IL-2, CCL2, CCL3, CCL4, CCL5, CCL8, CCL9, CCL10, CCL11 , CXCL8, CXCL9, CXCL10, CXCL16, stem cell factor (SCF), histidine-rich glycoprotein, paclitaxel, SOCS3, Activin A, BtK, iNOS and/or NOS2.

8. The phagocytic cell according to any one of claims 1 to 7, wherein the cargo protein comprises one or more detectable agents, wherein optionally the one or more detectable agents are fluorescent and/or bioluminescent agents.

9. A population comprising more than 1 × 10⁵ phagocytic cells according to any one of claims 1 to 8, wherein optionally the population is autologous.

10. A package comprising a phagocytic cell according to any one of claims 1 to 8 or a population according to claim 9.

11. A method of producing a phagocytic cell according to any one of claims 1 to 8 or a population of phagocytic cells according to claim 9, comprising:
(a) contacting the one or more phagocytic cells with polyhedrin protein crystals; and
(b) culturing the phagocytic cells under conditions which induce phagocytosis, wherein optionally:
(i) the polyhedrin protein crystals are attached to a solid support prior to being contacted with the phagocytic cells; or
(ii) the phagocytic cells are mixed with polyhedrin protein crystals prior to culturing the phagocytic cells,
wherein optionally the method further comprises:
(c) isolating the one or more phagocytic cells; and/or
(d) formulating the one or more phagocytic cells for delivery via an injectable route.

12. A pharmaceutical composition comprising:
(a) a phagocytic cell according to any one of claims 1 to 8 or a population according to claim 9; and
(b) a pharmaceutically acceptable carrier or diluent.

13. The pharmaceutical composition according to claim 12 for use in medicine.

14. The pharmaceutical composition according to claim 12, for use in a method of treating and/or preventing a solid cancer in a subject.

15. A phagocytic cell or population thereof for use in a method of imaging a solid cancer, wherein the phagocytic cell is a professional cell and comprises one or more polyhedrin protein crystals wherein the crystals encapsulate one or more detectable agents, or the population comprises more than 1 × 10⁵ phagocytic cells, and wherein the method comprises:
(a) administering the phagocytic cell or the population thereof; and
(b) detecting the detectable agent, thereby imaging at least part of the cancer,
wherein optionally the detectable agent is a fluorescent or luminescent agent.

## Patentansprüche

1. Phagozytenzelle, umfassend ein oder mehrere Polyhedrinproteinkristalle, wobei die Kristalle ein oder mehrere Frachtproteine einkapseln, wobei die Phagozytenzelle eine professionelle Zelle ist und das Frachtprotein ein oder mehrere therapeutische Mittel umfasst.

2. Phagozytenzelle nach Anspruch 1, wobei:
(a) die Kristalle in die Phagozytenzelle aufgenommen werden,
(b) die Kristalle dazu in der Lage sind, die Frachtproteine intakt innerhalb der Phagozytenzelle auszuscheiden; und/oder
(c) die Frachtproteine dazu in der Lage sind, nachhaltig aus der Phagozytenzelle freigesetzt zu werden,
wobei die Zellen optional Säugetierzellen sind, wobei die Säugetierzellen ferner optional menschliche Zellen sind.

3. Phagozytenzelle nach Anspruch 1 oder 2, wobei die Kristalle kleiner als 10 Mikrometer und/oder im Wesentlichen kubisch geformt sind, wobei optional:
(a) das Polyhedrinprotein die Polyhedrinsequenz des Bombyx-mori-Cypovirus umfasst, wie in SEQ ID NO:1 dargelegt; oder
(b) das Polyhedrinprotein eine Sequenz umfasst, die mindestens 70 % Homologie zu der Sequenz von SEQ ID NO:1 auf Grundlage einer Aminosäureidentität über die gesamte Sequenz von SEQ ID NO:1 aufweist,
wobei die Frachtproteine ferner optional eine oder mehrere Polyhedrin-bindende Tag-Sequenzen umfassen, die aus Folgenden ausgewählt sind:
(a) der H1-Tag-Sequenz wie in SEQ ID NO:2 dargelegt;
(b) der VP3-Tag-Sequenz wie in SEQ ID NO:3 dargelegt;
(c) der PH-Tag-Sequenz wie in SEQ ID NO:4 dargelegt; oder
(d) einer Sequenz, die mindestens 70 % Homologie zu der Sequenz von jeweils SEQ ID NO:2, 3 oder 4 auf Grundlage einer Aminosäureidentität über die gesamte Sequenz von SEQ ID NO:2, 3 oder 4 aufweist.

4. Phagozytenzelle nach einem der Ansprüche 1 bis 3, wobei die professionelle Zelle ein Monozyt, Makrophage oder eine Gliazelle ist, wobei die Phagozytenzelle optional ein Makrophage ist.

5. Phagozytenzelle nach einem der Ansprüche 1 bis 4, wobei die Phagozytenzelle aus dem peripheren Blut eines Subjekts stammt.

6. Phagozytenzelle nach einem der Ansprüche 1 bis 5, wobei das eine oder die mehreren therapeutischen Mittel dazu in der Lage sind, auf eine Zelle, ein extrazelluläres Vesikel oder ein freies Protein in einer Tumormikroumgebung abzuzielen.

7. Phagozytenzelle nach Anspruch 6, wobei:
(a) das eine oder die mehreren therapeutischen Mittel dazu in der Lage sind, die Herstellung von M1-Makrophagenzellen zu stimulieren; und/oder
(b) das eine oder die mehreren therapeutischen Mittel dazu in der Lage sind, M2-tumorassoziierte Makrophagen (TAMS), entzündliche Monozyten und/oder myeloide Suppressorzellen (MDSC) in M1-Makrophagenzellen umzuprogrammieren,
wobei optional das eine oder die mehreren therapeutischen Mittel Wachstumsfaktoren, Zytokine, Enzyme, Signalmoleküle, Hormone, Antikörper und/oder Impfstoffe sind, wobei ferner optional das eine oder die mehreren therapeutischen Mittel aus Folgendem ausgewählt sind:
(a) Th-1-verwandten Zytokinen und/oder TLR-Liganden; und/oder
(b) einem Granulozyten-Makrophagen-Kolonie-stimulierenden Faktor (GM-CSF), CSF-2, CSF-1 (M-CSF), CSF-3 (G-CSF), Interferon-gamma (IFN-γ), TNF-α, IFN-β, IL-6, IL-12, IL-15, IL-21, IL-23, MHC-II, IL-1 R, TLR2, TLR4, CD80, CD86, CD68, IL-1, IL-1-beta, IL-2, CCL2, CCL3, CCL4, CCL5, CCL8, CCL9, CCL10, CCL11, CXCL8, CXCL9, CXCL10, CXCL16, einem Stammzellfaktor (SCF), einem histidinreichen Glykoprotein, Paclitaxel, SOCS3, Activin A, BtK, iNOS und/oder NOS2.

8. Phagozytenzelle nach einem der Ansprüche 1 bis 7, wobei das Frachtprotein ein oder mehrere nachweisbare Mittel umfasst, wobei es sich bei dem einen oder den mehreren nachweisbaren Mitteln optional um fluoreszierende und/oder biolumineszierende Mittel handelt.

9. Population, umfassend mehr als 1 × 10⁵ Phagozytenzellen nach einem der Ansprüche 1 bis 8, wobei die Population optional autolog ist.

10. Verpackung, umfassend eine Phagozytenzelle nach einem der Ansprüche 1 bis 8 oder eine Population nach Anspruch 9.

11. Verfahren zum Herstellen einer Phagozytenzelle nach einem der Ansprüche 1 bis 8 oder einer Population von Phagozytenzellen nach Anspruch 9, umfassend:
(a) Inkontaktbringen der einen oder mehreren Phagozytenzellen mit Polyhedrinproteinkristallen; und
(b) Kultivieren der Phagozytenzellen unter Bedingungen, die eine Phagozytose auslösen, wobei optional:
(i) die Polyhedrinproteinkristalle vor dem Inkontaktbringen mit den Phagozytenzellen an einem festen Träger angebracht werden, oder
(ii) die Phagozytenzellen vor dem Kultivieren der Phagozytenzellen mit Polyhedrinproteinkristallen vermischt werden,
wobei das Verfahren optional ferner Folgendes umfasst:
(c) Isolieren der einen oder mehreren Phagozytenzellen; und/oder
(d) Formulieren der einen oder mehreren Phagozytenzellen zur Abgabe über einen injizierbaren Weg.

12. Pharmazeutische Zusammensetzung, umfassend:
(a) eine Phagozytenzelle nach einem der Ansprüche 1 bis 8 oder eine Population nach Anspruch 9; und
(b) einen pharmazeutisch unbedenklichen Träger oder Verdünner.

13. Pharmazeutische Zusammensetzung nach Anspruch 12 zur Verwendung in der Medizin.

14. Pharmazeutische Zusammensetzung nach Anspruch 12 zur Verwendung in einem Verfahren zum Behandeln und/oder Vorbeugen eines soliden Krebses bei einem Subjekt.

15. Phagozytenzelle oder Population davon zur Verwendung in einem Verfahren zum Abbilden eines soliden Krebses, wobei die Phagozytenzelle eine professionelle Zelle ist und ein oder mehrere Polyhedrinproteinkristalle umfasst, wobei die Kristalle ein oder mehrere nachweisbare Mittel einkapseln oder die Population mehr als 1 × 10⁵ Phagozytenzellen umfasst und wobei das Verfahren Folgendes umfasst:
(a) Verabreichen der Phagozytenzelle oder der Population davon; und
(b) Nachweisen des nachweisbaren Mittels und dadurch Abbilden von mindestens einem Teil des Krebses,
wobei das nachweisbare Mittel optional ein fluoreszierendes oder lumineszierendes Mittel ist.

## Revendications

1. Cellule phagocytaire comprenant un ou plusieurs cristaux de protéine polyhédrine, dans laquelle les cristaux encapsulent une ou plusieurs protéines cargo, la cellule phagocytaire étant une cellule professionnelle et la protéine cargo comprenant un ou plusieurs agents thérapeutiques.

2. Cellule phagocytaire selon la revendication 1, dans laquelle :
(a) les cristaux sont internalisés dans la cellule phagocytaire ;
(b) les cristaux sont capables de sécréter les protéines cargo dans la cellule phagocytaire intacte ; et/ou
(c) les protéines cargo peuvent être libérées durablement de la cellule phagocytaire,
les cellules étant éventuellement des cellules de mammifères, encore éventuellement, les cellules de mammifères étant des cellules humaines.

3. Cellule phagocytaire selon la revendication 1 ou 2, dans laquelle les cristaux sont de taille inférieure à 10 microns et/ou de forme sensiblement cubique, dans laquelle, éventuellement :
(a) la protéine polyhédrine comprend la séquence de polyhédrine de cypovirus de Bombyx mori, comme indiqué dans ID SEQ NO:1 ; ou
(b) la protéine polyhédrine comprend une séquence qui a au moins 70 % d'homologie avec la séquence de SEQ ID NO: 1 basée sur l'identité des acides aminés sur toute la séquence de SEQ ID NO: 1,
dans laquelle, encore éventuellement, les protéines cargo comprennent une ou plusieurs séquences étiquettes de liaison à la polyhédrine choisies parmi :
(a) la séquence H1-tag telle qu'indiquée dans SEQ ID NO: 2 ;
(b) la séquence VP3-tag telle qu'indiquée dans SEQ ID NO: 3 ;
(c) la séquence PH tag telle qu'indiquée dans SEQ ID NO: 4 ; ou
(d) une séquence qui a au moins 70 % d'homologie avec la séquence de SEQ ID NO : 2, 3 ou 4 basée sur l'identité des acides aminés sur l'ensemble de la séquence de SEQ ID NO: 2, 3 ou 4 respectivement.

4. Cellule phagocytaire selon l'une quelconque des revendications 1 à 3, dans laquelle la cellule professionnelle est un monocyte, un macrophage ou une cellule gliale, la cellule phagocytaire étant éventuellement un macrophage.

5. Cellule phagocytaire selon l'une quelconque des revendications 1 à 4, la cellule phagocytaire étant dérivée du sang périphérique d'un sujet.

6. Cellule phagocytaire selon l'une quelconque des revendications 1 à 5, dans laquelle un ou plusieurs agents thérapeutiques sont capables de cibler une cellule, une vésicule extracellulaire ou une protéine libre dans un microenvironnement tumoral.

7. Cellule phagocytaire selon la revendication 6, dans laquelle :
(a) le ou les plusieurs agents thérapeutiques sont capables de stimuler la production de cellules macrophages M1 ; et/ou
(b) le ou les plusieurs agents thérapeutiques sont capables de reprogrammer les macrophages M2 associés à la tumeur (TAMS), les monocytes inflammatoires et/ou les cellules suppressives dérivées des myéloïdes (MDSC) en cellules macrophages M1,
dans laquelle, éventuellement le ou les plusieurs agents thérapeutiques sont des facteurs de croissance, des cytokines, des enzymes, des molécules de signalisation, des hormones, des anticorps et/ou des vaccins, éventuellement, un ou plusieurs agents thérapeutiques étant choisis parmi :
(a) les cytokines apparentées Th-1 et/ou les ligands TLR ; et/ou
(b) le facteur de stimulation des colonies de granulocytes et de macrophages (GM-CSF), CSF-2, CSF-1 (M-CSF), CSF-3 (G-CSF), interféron gamma (IFN-γ), TNF-α, IFN-β, IL-6, IL-12, IL-15, IL-21, IL-23, MHC-II, IL-1 R, TLR2, TLR4, CD80, CD86, CD68, IL-1 , IL-1 bêta, IL-2, CCL2, CCL3, CCL4, CCL5, CCL8, CCL9, CCL10, CCL11 , CXCL8, CXCL9, CXCL10, CXCL16, facteur des cellules souches (SCF), glycoprotéine riche en histidine, paclitaxel, SOCS3, Activine A, BtK, INOS et/ou NOS2.

8. Cellule phagocytaire selon l'une quelconque des revendications 1 à 7, dans laquelle la protéine cargo comprend un ou plusieurs agents détectables, dans laquelle, éventuellement, un ou plusieurs agents détectables sont des agents fluorescents et/ou bioluminescents.

9. Population comprenant plus de 1 × 10⁵ cellules phagocytaires selon l'une quelconque des revendications 1 à 8, la population étant éventuellement autologue.

10. Ensemble comprenant une cellule phagocytaire selon l'une quelconque des revendications 1 à 8 ou une population selon la revendication 9.

11. Procédé de production d'une cellule phagocytaire selon l'une quelconque des revendications 1 à 8 ou d'une population de cellules phagocytaires selon la revendication 9, comprenant :
(a) la mise en contact de la ou des plusieurs cellules phagocytaires avec des cristaux de protéines polyhédrine ; et
(b) la culture des cellules phagocytaires dans des conditions qui induisent une phagocytose, dans lesquelles, éventuellement :
(i) les cristaux de protéine polyhédrine sont fixés sur un support solide avant d'être mis en contact avec les cellules phagocytaires ; ou
(II) les cellules phagocytaires sont mélangées à des cristaux de protéine polyhédrine avant la culture des cellules phagocytaires,
éventuellement le procédé comprenant en outre :
(c) l'isolement de la ou des plusieurs cellules phagocytaires ; et/ou
(d) la formulation de la ou des plusieurs cellules phagocytaires pour l'administration par une voie injectable.

12. Composition pharmaceutique comprenant :
(a) une cellule phagocytaire selon l'une quelconque des revendications 1 à 8 ou une population selon la revendication 9 ; et
(b) un véhicule ou diluant pharmaceutiquement acceptable.

13. Composition pharmaceutique selon la revendication 12 destinée à être utilisée en médecine.

14. Composition pharmaceutique selon la revendication 12 destinée à être utilisée dans une méthode de traitement et/ou de prévention d'un cancer solide chez un sujet.

15. Cellule phagocytaire ou population de celle-ci destinée à être utilisée dans une méthode d'imagerie d'un cancer solide, la cellule phagocytaire étant une cellule professionnelle et comprenant un ou plusieurs cristaux de protéine polyhédrine, les cristaux encapsulant un ou plusieurs agents détectables, ou la population comprenant plus de 1 × 10⁵ cellules phagocytaires, et la méthode comprenant :
(a) l'administration de la cellule phagocytaire ou de la population de celle-ci ; et
(b) la détection de l'agent détectable, réalisant ainsi l'imagerie d'au moins une partie du cancer,
dans lequel éventuellement, l'agent détectable étant un agent fluorescent ou luminescent.
